# EUROPEAN PATENT APPLICATION

(11) **EP 2 042 140 A1**
(43) Date of publication of application: **01.04.2009**
(21) Application number: 07768254.0
(22) Date of filing: 05.07.2007
(51) Int. Cl.: A61F 13/15, A61F 13/472, A61F 13/534

(54) **ABSORPTIVE ARTICLE**

(30) Priority: 05.07.2006 JP 2006186140
(71) Applicant: Uni-Charm Corporation, Ehime 799-0111 (JP)
(72) Inventor: KUDO, Jun, Kanonji-shi Kagawa 769-1602 (JP); HASHINO, Akira c/o Technical Center, Kanonji-shi Kagawa 769-1602 (JP)
(74) Representative: Sperling, Rüdiger
(86) International application number: PCT/JP2007/063506
(87) International publication number: WO 2008/004638

(57) **Abstract**

Provided is an absorptive article having absorption elements capable of following the body of a user. This absorptive article (1) comprises a generally rectangular base absorption element (2), a top absorption element (3) disposed on one surface of the base absorption element (2) at substantially lateral center of the base absorption element (2) and along the longitudinal direction of the base absorption element (2), and a fixing part (4) for fixing the base absorption element (2) to the top absorption element (3) so that at least one end of the top absorption element (3) in the longitudinal direction thereof is made to be a free end (31). The flexural rigidity and the compression hardness of the top absorption element (3) are set within appropriate ranges, respectively.

## Description

### TECHNICAL FIELD

The present invention relates to an absorbent article.

### BACKGROUND ART

Conventionally, as an absorbent article for absorbing a predetermined liquid such as menstrual blood, an absorbent article that is entirely formed in a sheet-like shape, including an absorbent layer for absorbing the predetermined liquid, a liquid permeable top sheet for covering a skin contacting surface of the absorbent layer, and a liquid impermeable back surface sheet for covering a clothing side surface of the absorbent layer, can be exemplified. Such an absorbent article, which is substantially sheet-shaped, is used in contact with an excretory part and directly absorbs the liquid such as menstrual blood discharged from the excretory part. Various improvements have been made thereto in order to prevent menstrual blood and the like, which runs along a predetermined groove of a wearer's body, from contacting clothing and the like.

For example, a means for preventing leakage of menstrual blood includes wings projecting in a width direction, provided on both sides of an absorbent article, for fixing the absorbent article by being folded back toward underwear, and for raising an absorbent core in the vicinity of excretory part of menstrual blood toward the excretory part in order to make the absorbent core adhere to the excretory part in order to absorb menstrual blood. However, such absorbent articles may not appropriately follow movement of a wearer's body and change in body surface shape due to movement.

In contrast, an absorbent article is proposed including a lower absorbent core fixed to an absorbent article main body and an upper absorbent core independent therefrom, in which an elastic, stretchable intermediate sheet disposed in a longitudinal direction between the upper absorbent core and the lower absorbent core pushes up the upper absorbent core and makes the upper absorbent core adhere to a wearer's body, in order to improve followability with respect to an excretory part (for example, see Patent Document 1).

Patent Document 1: Japanese Unexamined Patent Application No. 2000-152957

### DISCLOSURE OF THE INVENTION

### Problems to be Solved by the Invention

However, an absorbent article in Patent Document 1 has an uppermost surface thereof which becomes wide in the width direction and forming a plane when worn because an upper absorbent core is wholly fixed along the longitudinal direction to a lower absorbent core of a sanitary napkin and is fixed in the width direction at both ends of the lower absorbent core. Therefore, a predetermined space is produced between the upper absorbent core and a groove of the wearer's body. Therefore, the upper absorbent core cannot come into contact with and tightly fit the excretion area in an inner part of the groove of the wearer's body without any clearance, so that menstrual blood may leak.

Furthermore, the upper absorbent core can follow slight lateral movement of the wearer's body by being deformed. However, the upper absorbent core cannot follow such movement of the wearer's body or underwear that it is greatly dislocated in the width direction and longitudinal movement of the wearer's body or underwear.

In addition, since the upper absorbent core is completely fixed to the lower absorbent core along the longitudinal direction, when a user wears the sanitary napkin with the sanitary napkin attached to the underwear, the sanitary napkin is worn at a position where it cannot be seen by the user. Thus, the user cannot always wear the sanitary napkin such that the upper absorbent core sufficiently fits tightly with the groove of the wearer's body. In a case where the user cannot wear the sanitary napkin so as to tightly fit the groove of the wearer's body, a predetermined space is produced between the upper absorbent core and the excretory part so that the menstrual blood may leak.

The present invention has been made in view of the foregoing problems and has an objective of providing an absorbent article that includes an absorbent core capable of following the wearer's body.

### Means for Solving the Problems

In a first aspect of the present invention, an absorbent article includes: a substantially elongated first absorbent core (for example, a base absorbent core 2 in a first embodiment described later); a second absorbent core (for example, a top absorbent core 3 in the first embodiment described later) disposed on a skin contacting side of the first absorbent core, along a longitudinal direction of the first absorbent core; and a fixing portion for fixing the first absorbent core with the second absorbent core so that at least one end edge in a longitudinal direction of the second absorbent core is made to be a free end, in which a flexural rigidity of the second absorbent core toward a side that is opposite to a side facing the first absorbent core is 0.05 to 1.7 N.

According to a second aspect of the present invention, in the absorbent article as described in the first aspect, the flexural rigidity of a central region, which is a region in the second absorbent core from a position to be in contact with an excretory part to a position being 100 mm spaced apart from the one end edge of the second absorbent core, is 0.1 to 1.5 N.

According to a third aspect of the present invention, in the absorbent article as described in the first or the second aspect, a difference in the flexural rigidity between a predetermined position on the second absorbent core and another position spaced apart therefrom in the longitudinal direction is no greater than 1.2 N.

According to a fourth aspect of the present invention, in the absorbent article as described in any one of the first to the third aspects, a value of the flexural rigidity of the second absorbent core is 0.1 to 2 times greater than a value of the flexural rigidity of the first absorbent core toward the second absorbent core.

In a fifth aspect of the present invention, an absorbent article includes: a substantially elongated first absorbent core; a second absorbent core disposed on one side of the first absorbent core, along a longitudinal direction of the first absorbent core; and a fixing portion for fixing the first absorbent core with the second absorbent core so that at least one end edge in a longitudinal direction of the second absorbent core is made to be a free end, in which compression hardness (LC) of a surface of the second absorbent core that is opposite to a side facing the first absorbent core is 0 to 0.8 (-).

According to a sixth aspect of the present invention, in the absorbent article as described in the fifth aspect, the compression hardness (Linearity of Compression) of a central region, which is a region in the second absorbent core from a position to be in contact with an excretory part to a position being 100 mm spaced apart from the one end edge of the second absorbent core, is 0.3 to 0.75 (-).

According to a seventh aspect of the present invention, in the absorbent article as described in the fifth or the sixth aspect, a value of the compression hardness (LC) of the second absorbent core is 0.1 to 2 times greater than a value of the compression hardness (LC) of the first absorbent core.

In an eighth aspect of the present invention, an absorbent article includes: a substantially elongated first absorbent core; a second absorbent core disposed on one side of the first absorbent core, along a longitudinal direction of the first absorbent core; and a fixing portion for fixing the first absorbent core with the second absorbent core so that at least one end edge in a longitudinal direction of the second absorbent core is made to be a free end, in which: the second absorbent core has a first region that is formed substantially in a center in a width direction, which is a direction orthogonal to the longitudinal direction of the second absorbent core, extending in the longitudinal direction; and rigidity of the first region is different from rigidity of second regions that are formed on both sides of the first region in the width direction, extending in the longitudinal direction.

In a ninth aspect of the present invention, an absorbent article includes: a substantially elongated first absorbent core; a second absorbent core disposed on one face of the first absorbent core, along a longitudinal direction of the first absorbent core; and a fixing portion that fixes the first absorbent core with the second absorbent core so that at least one end edge in a longitudinal direction of the second absorbent core is made to be a free end, in which: the second absorbent core includes a second absorbent layer having liquid retention properties; and a predetermined fold starting point element is formed substantially in a center in a width direction, which is a direction orthogonal to the longitudinal direction of the second absorbent core, along the longitudinal direction of the second absorbent core.

According to a tenth aspect of the present invention, in the absorbent article of the ninth aspect, the second absorbent layer is formed by including a hydrophilic fiber; and in the fold starting point element, a basis weight of a first region is formed to be continuous or intermittent so as to extend in the longitudinal direction, substantially in a center in the width direction of the second absorbent layer, the basis weight in the first region is different from basis weight of second regions, which are formed on both sides in the width direction of the first region so as to extend in the longitudinal direction.

In an eleventh aspect of the present invention, an absorbent article includes: a substantially elongated first absorbent core; a second absorbent core disposed on a first face of the first absorbent core, along a longitudinal direction of the first absorbent core; and a fixing portion that fixes the first absorbent core with the second absorbent core so that at least one end edge in a longitudinal direction of the second absorbent core is made to be a free end, in which a core portion is disposed substantially in a center in a width direction, which is a direction orthogonal to the longitudinal direction of the second absorbent core, along the longitudinal direction of the second absorbent core.

According to a twelfth aspect of the present invention, in the absorbent article of the eleventh aspect, the core portion is a compressed portion formed to be continuous or intermittent along the longitudinal direction of the second absorbent core, substantially in a center in the width direction of the second absorbent core.

### Effects of the Invention

According to the present invention, an absorbent article can be provided that includes an absorbent core capable of following the wearer's body.

### BRIEF DESCRIPTION OF THE DRAWINGS

FIG. 1 is a plan view showing an absorbent article according to a first embodiment of the present invention;
FIG. 2 is a back view showing the absorbent article according to the first embodiment of the present invention;
FIG. 3A is a cross-sectional view of FIG. 1 of the absorbent article according to the first embodiment of the present invention;
FIG. 3B is a cross-sectional view of FIG. 1 of the absorbent article according to the first embodiment of the present invention;
FIG. 3C is a cross-sectional view of FIG. 1 of the absorbent article according to the first embodiment of the present invention;
FIG. 4 is a cross-sectional view taken along the line Y-Y of the absorbent article according to the first embodiment of the present invention;
FIG. 5 is a diagram showing an absorbent core disposed in a top absorbent core according to the first embodiment of the present invention;
FIG. 6 is a diagram showing an absorbent core disposed in a base absorbent core according to the first embodiment of the present invention;
FIG. 7 is a diagram showing a compressed groove of the base absorbent core according to the first embodiment of the present invention;
FIG. 8 is a perspective view of the absorbent article in a worn state according to the first embodiment of the present invention;
FIG. 9 is a perspective view of the absorbent article in a worn state according to the first embodiment of the present invention;
FIG. 10 is a perspective view showing a worn state of the absorbent article according to the first embodiment of the present invention;
FIG. 11 is a plan view showing a top absorbent core according to the first embodiment of the present invention;
FIG. 12 is a perspective view showing the top absorbent core according to the first embodiment of the present invention;
FIG. 13 is a perspective cross-sectional view showing a top absorbent core of the absorbent article according to the first embodiment of the present invention;
FIG. 14A is a cross-sectional view showing embodiments of the top absorbent core of the absorbent article according to the first embodiment of the present invention;
FIG. 14B is a cross-sectional view showing embodiments of the top absorbent core of the absorbent article according to the first embodiment of the present invention;
FIG. 14C is a cross-sectional view showing embodiments of the top absorbent core of the absorbent article according to the first embodiment of the present invention;
FIG. 14D is a cross-sectional view showing embodiments of the top absorbent core of the absorbent article according to the first embodiment of the present invention;
FIG. 14E is a cross-sectional view showing embodiments of the top absorbent core of the absorbent article according to the first embodiment of the present invention;
FIG. 15A is a plan view of a base absorbent core and a back view of a top absorbent core according to a second embodiment of the present invention;
FIG. 15B is a plan view of a base absorbent core and a back view of a top absorbent core according to the second embodiment of the present invention;
FIG. 16A is a plan view of a base absorbent core and a back view of a top absorbent core according to a third embodiment of the present invention;
FIG. 16B is a plan view of a base absorbent core and a back view of a top absorbent core according to the third embodiment of the present invention;
FIG. 17A is a plan view of a base absorbent core and a back view of a top absorbent core according to a fourth embodiment of the present invention;
FIG. 17B is a plan view of a base absorbent core and a back view of a top absorbent core according to the fourth embodiment of the present invention;
FIG. 18A is a plan view of a base absorbent core and a back view of a top absorbent core according to a fifth embodiment of the present invention;
FIG. 18B is a plan view of a base absorbent core and a back view of a top absorbent core according to the fifth embodiment of the present invention;
FIG. 19A is a back view of a base absorbent core and a plan view of a top absorbent core according to a sixth embodiment of the present invention; and
FIG. 19B is a back view of a base absorbent core and a plan view of a top absorbent core according to the sixth embodiment of the present invention.

### PREFERRED MODE FOR CARRYING OUT THE INVENTION

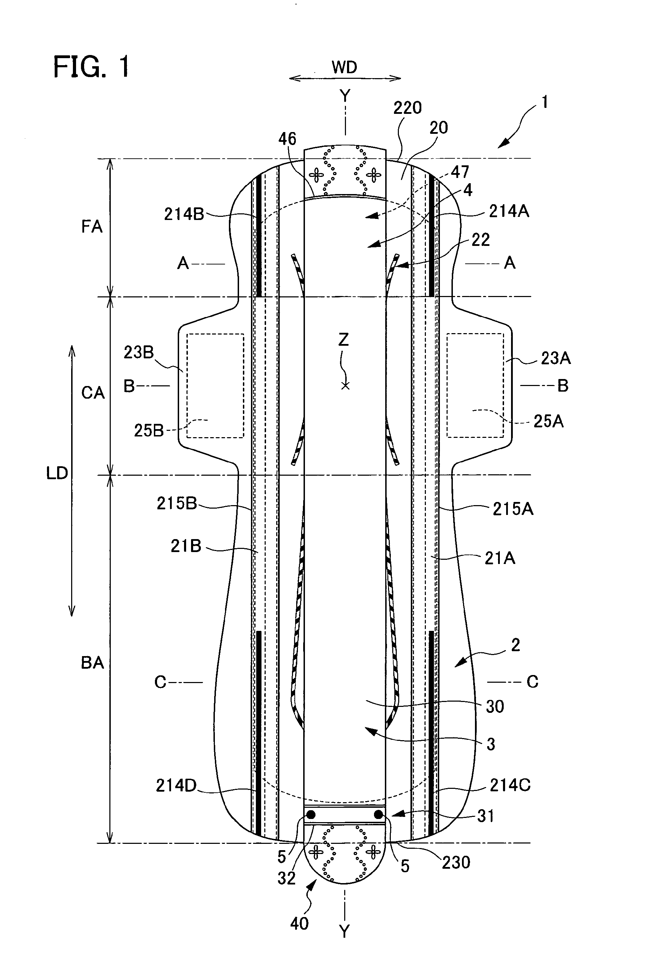

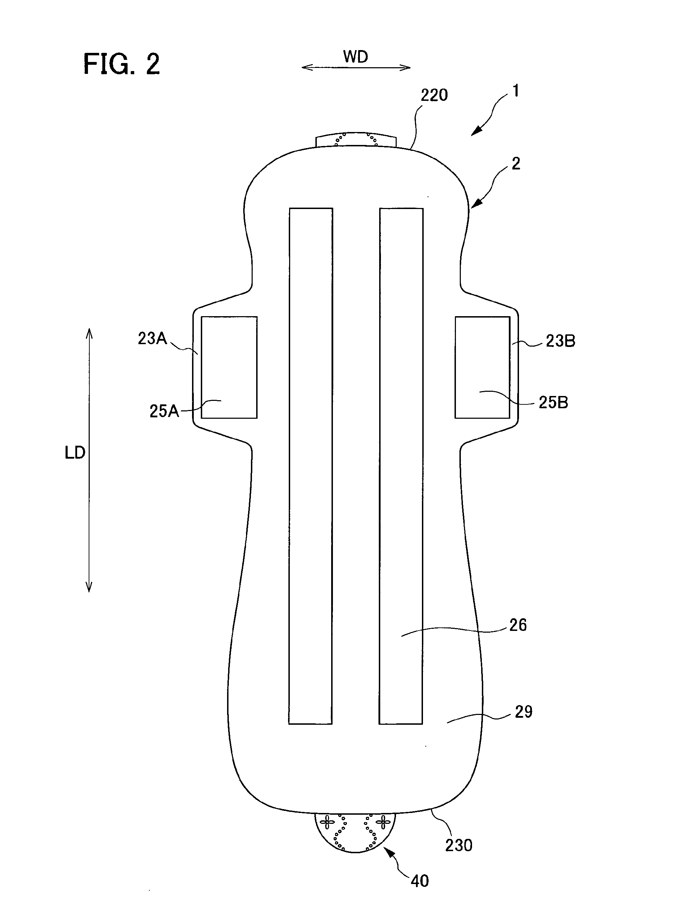

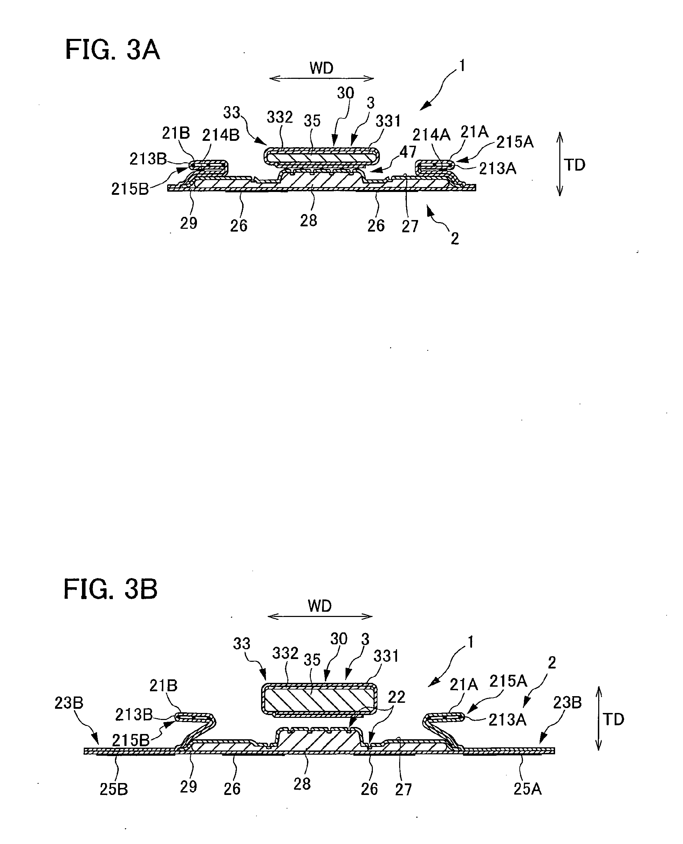

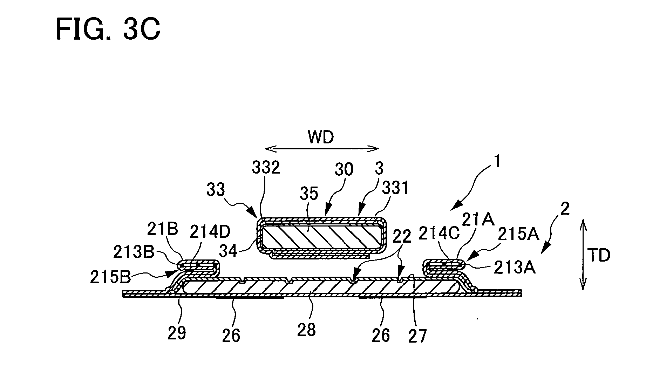

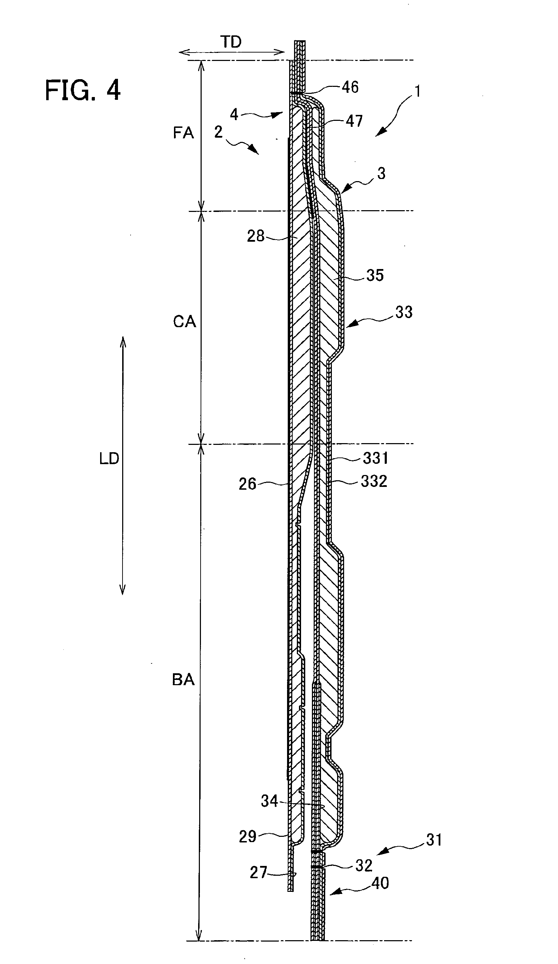

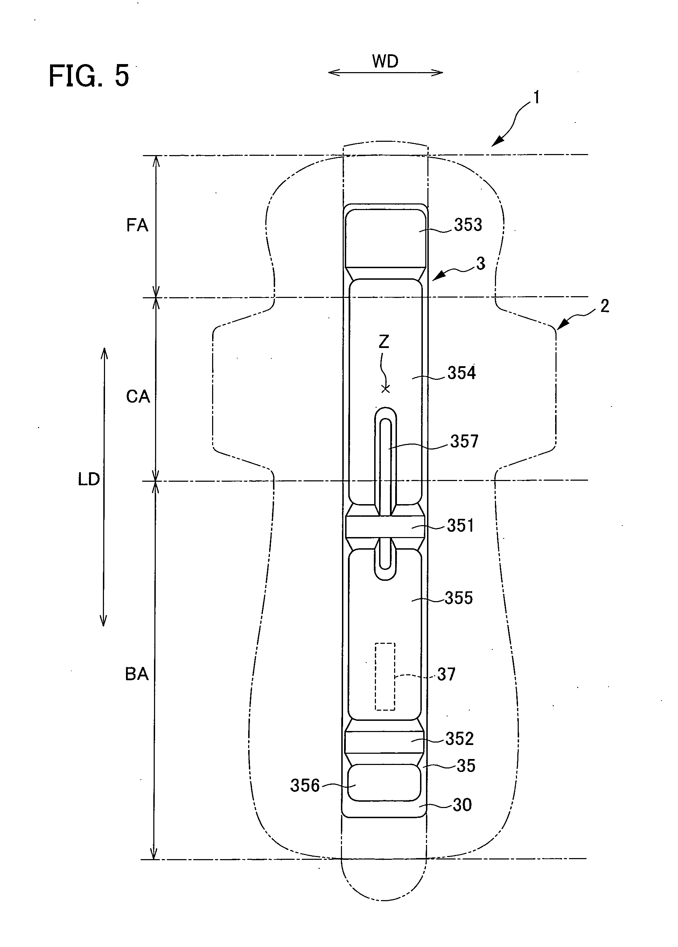

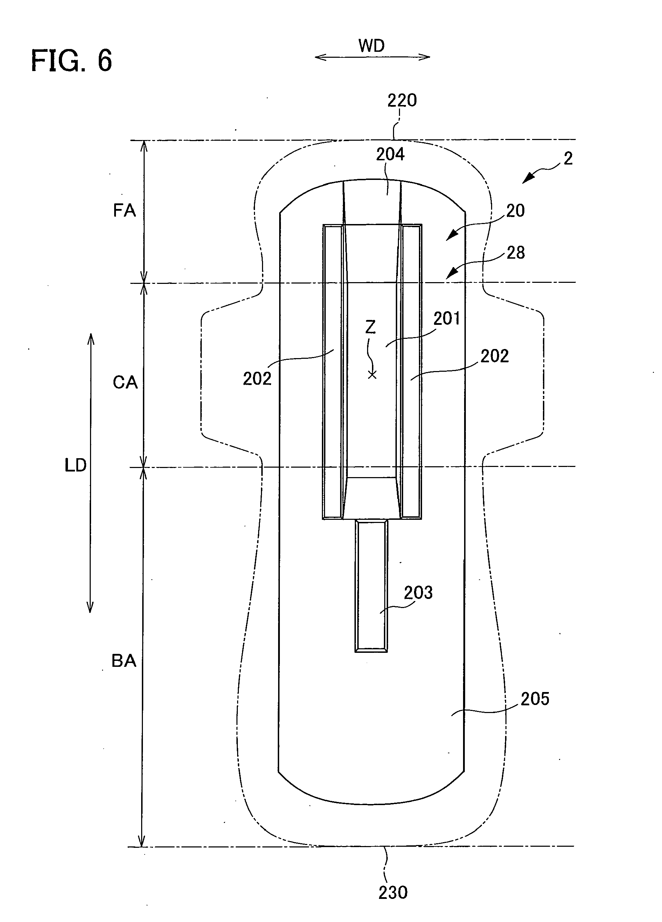

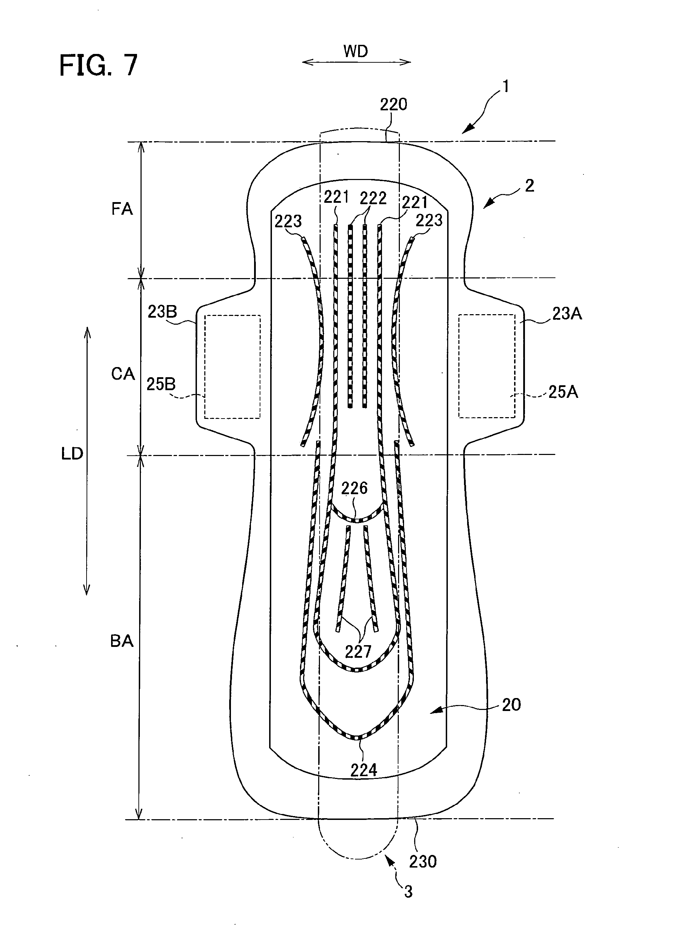

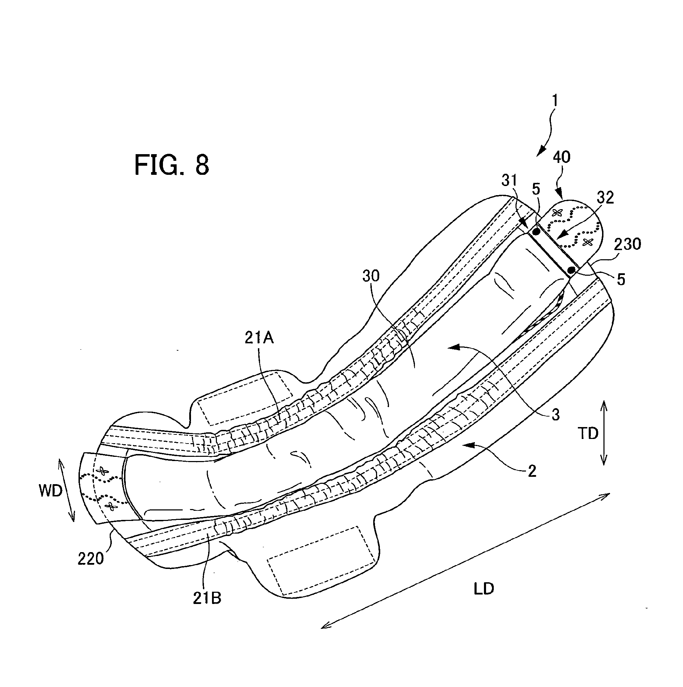

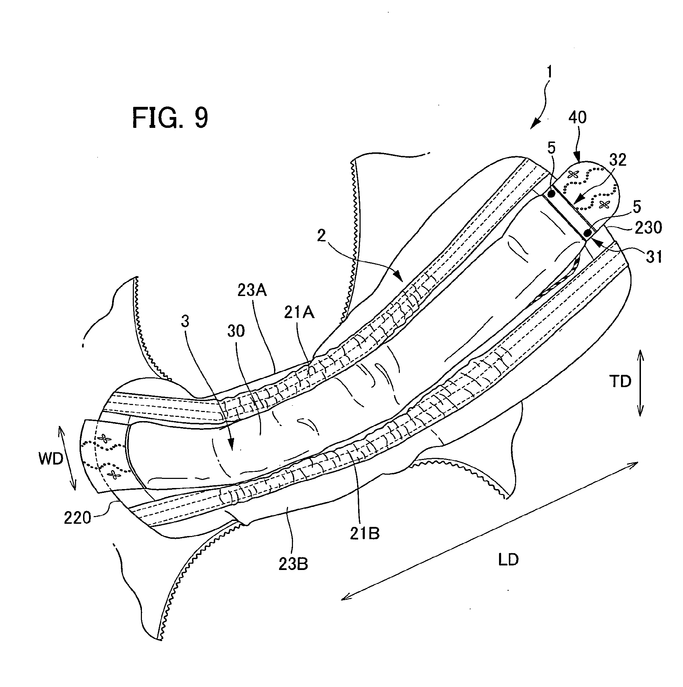

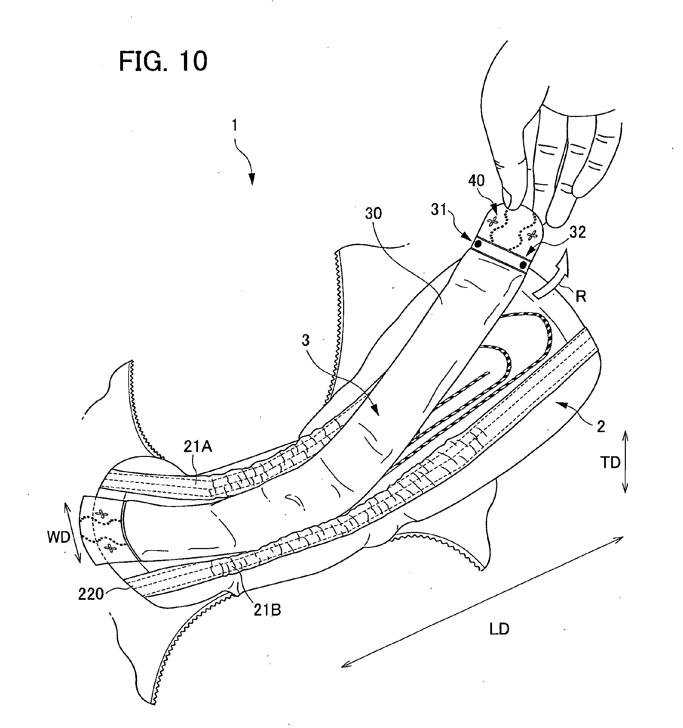

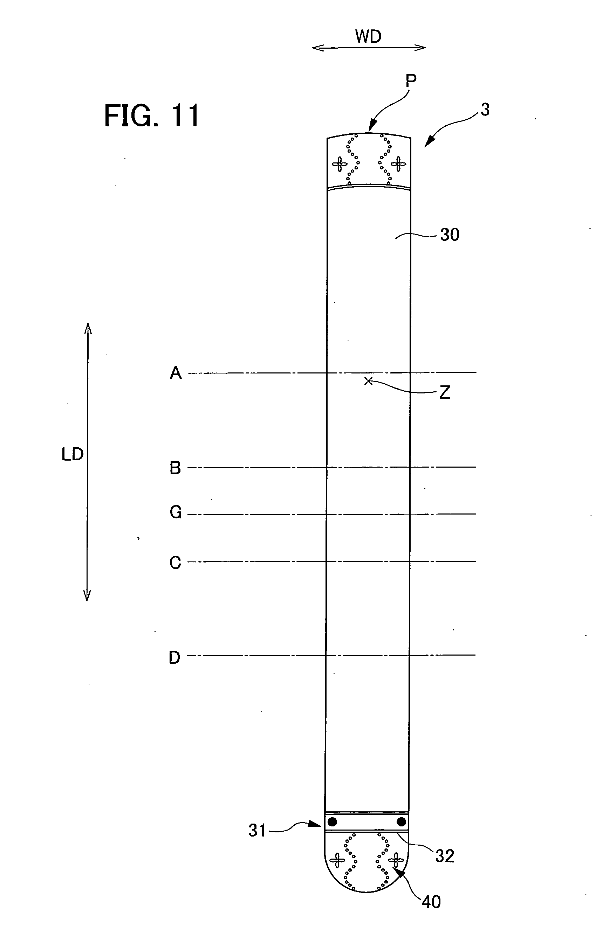

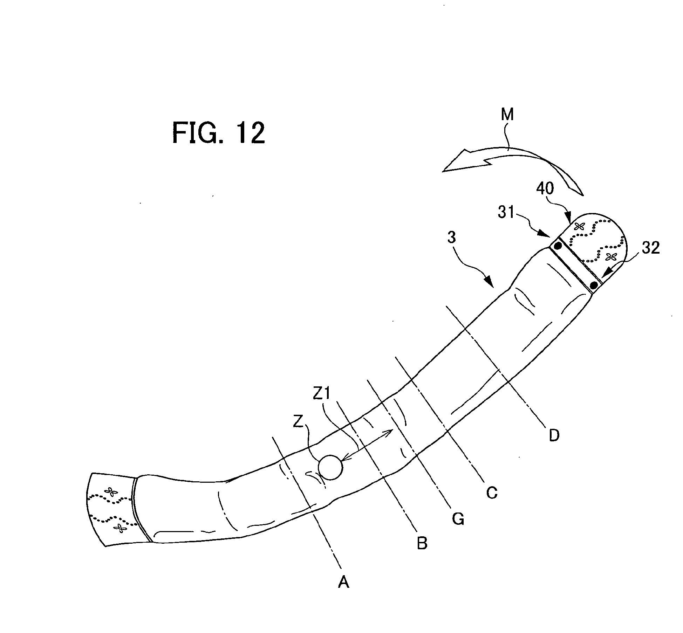

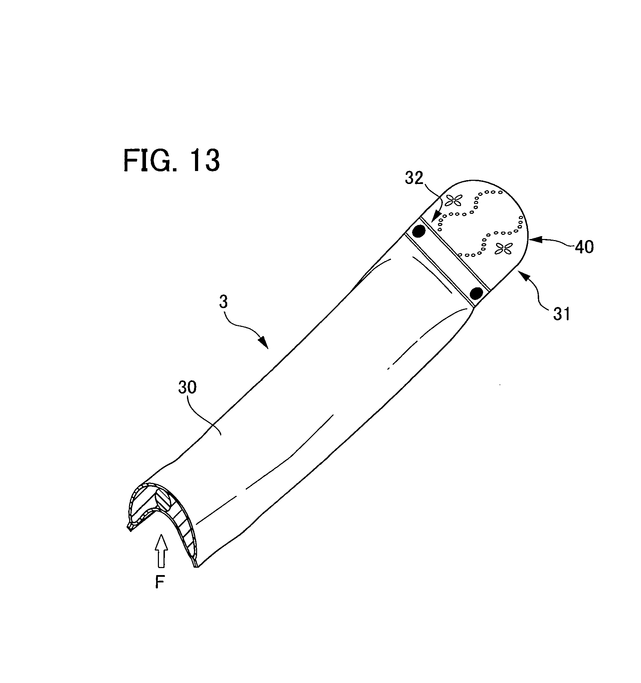

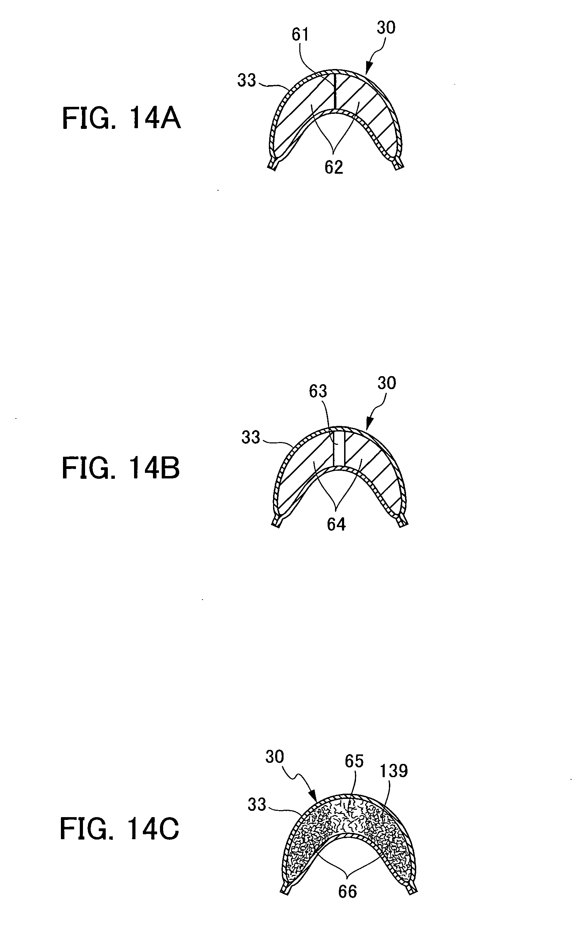

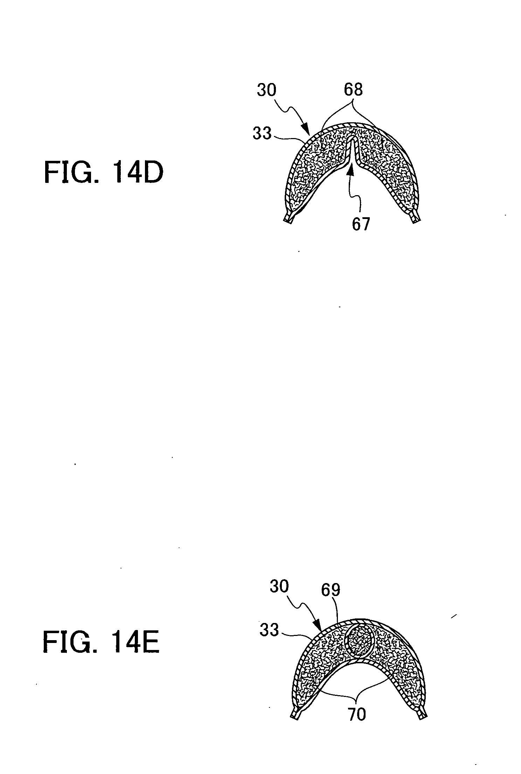

FIG. 1 is a plan view showing an absorbent article according to a first embodiment of the present invention. FIG. 2 is a back view showing the absorbent article according to the first embodiment of the present invention. FIG. 3A is a cross-sectional view of FIG. 1 of the absorbent article according to the first embodiment of the present invention. FIG. 3B is a cross-sectional view of FIG. 1 of the absorbent article according to the first embodiment of the present invention. FIG. 3C is a cross-sectional view of FIG. 1 of the absorbent article according to the first embodiment of the present invention. FIG. 4 is a cross-sectional view taken along the line Y-Y of the absorbent article according to the first embodiment of the present invention. FIG. 5 is a diagram showing an absorbent core disposed in a top absorbent core according to the first embodiment of the present invention. FIG. 6 is a diagram showing an absorbent core disposed in a base absorbent core according to the first embodiment of the present invention. FIG. 7 is a diagram showing a compressed groove of the base absorbent core according to the first embodiment of the present invention. FIG. 8 is a perspective view of the absorbent article in a worn state according to the first embodiment of the present invention. FIG. 9 is a perspective view of the absorbent article in a worn state according to the first embodiment of the present invention. FIG. 10 is a perspective view showing the absorbent article in a worn state according to the first embodiment of the present invention. FIG. 11 is a plan view showing a top absorbent core according to a first embodiment of the present invention. FIG. 12 is a perspective view showing a top absorbent core according to the first embodiment of the present invention. FIG. 13 is a perspective cross-sectional view showing a top absorbent core of the absorbent article according to the first embodiment of the present invention. FIG. 14A is a cross-sectional view showing embodiments of the top absorbent core of the absorbent article according to the first embodiment of the present invention. FIG. 14B is a cross-sectional view showing embodiments of the top absorbent core of the absorbent article according to the first embodiment of the present invention. FIG. 14C is a cross-sectional view showing embodiments of the top absorbent core of the absorbent article according to the first embodiment of the present invention. FIG. 14D is a cross-sectional view of the top absorbent core of the absorbent article according to the first embodiment of the present invention. FIG. 14E is a cross-sectional view of the top absorbent core of the absorbent article according to the first embodiment of the present invention.

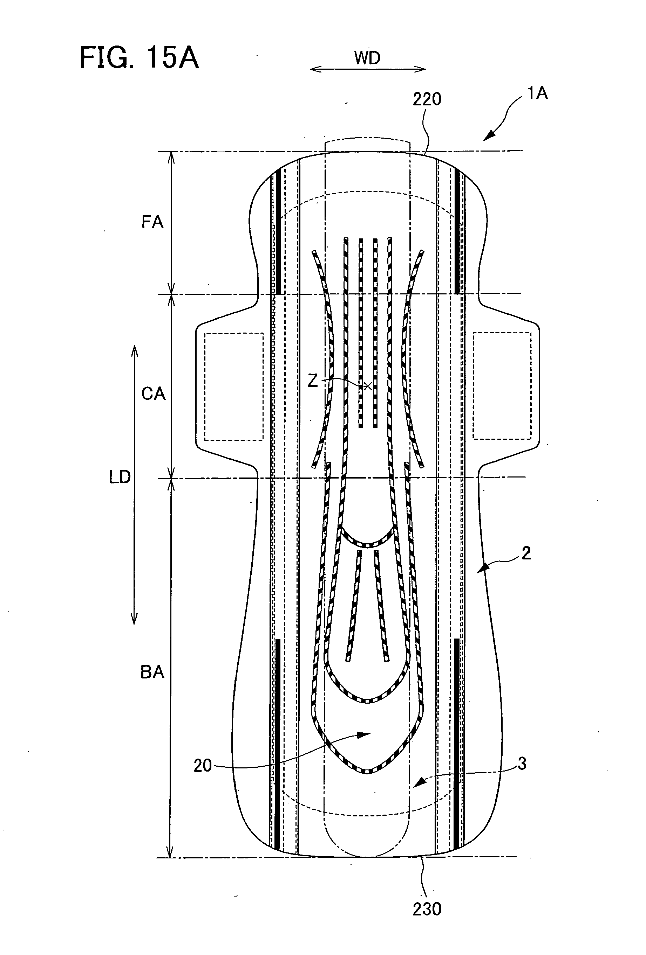

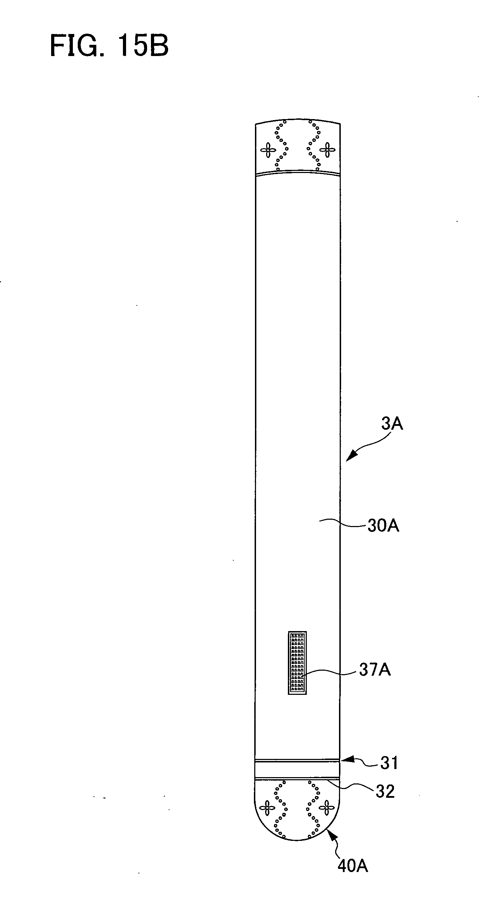

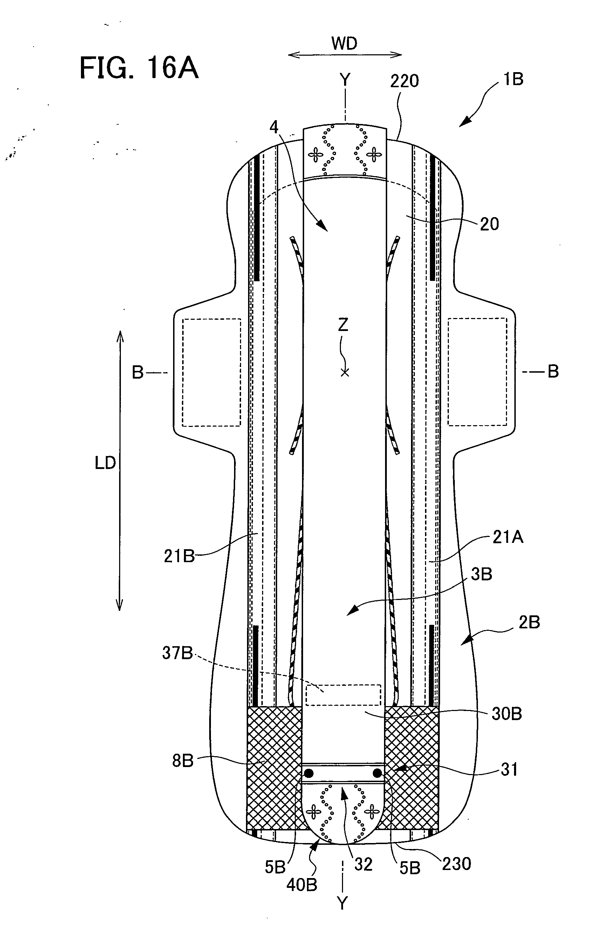

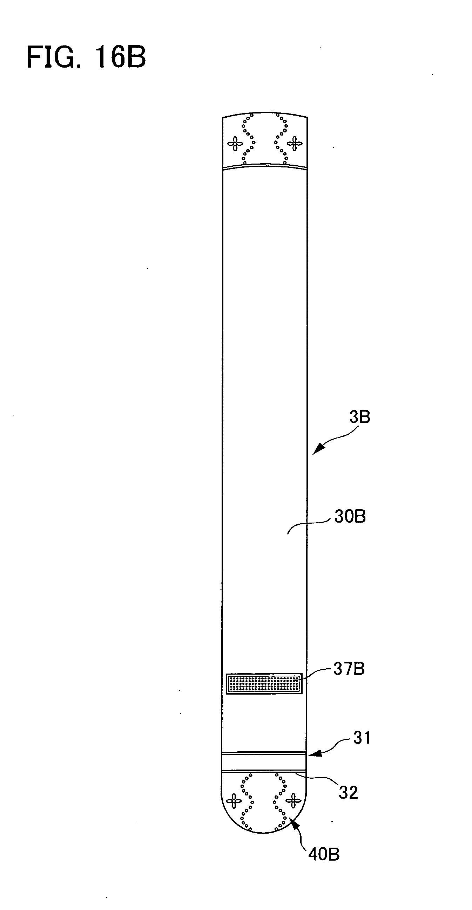

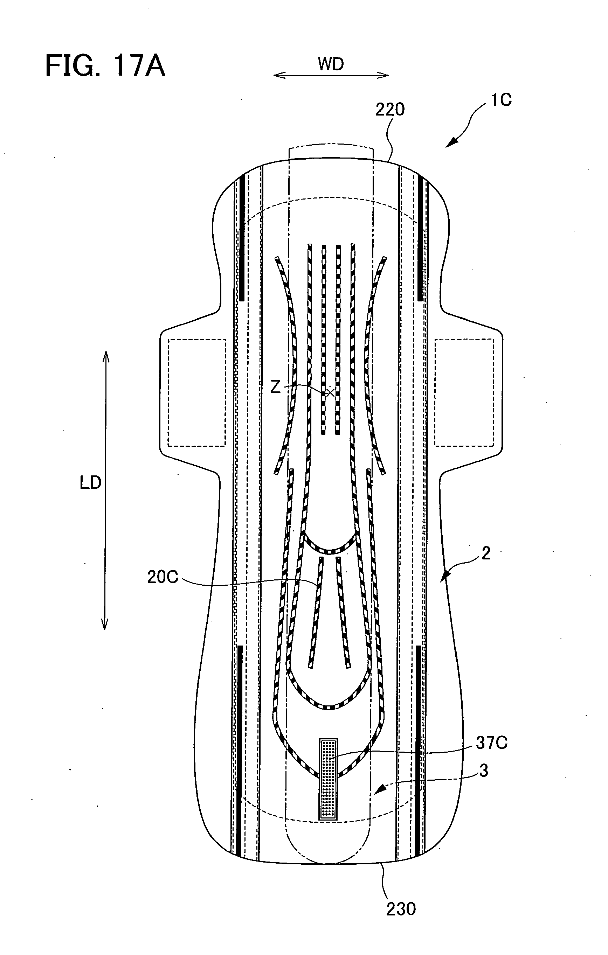

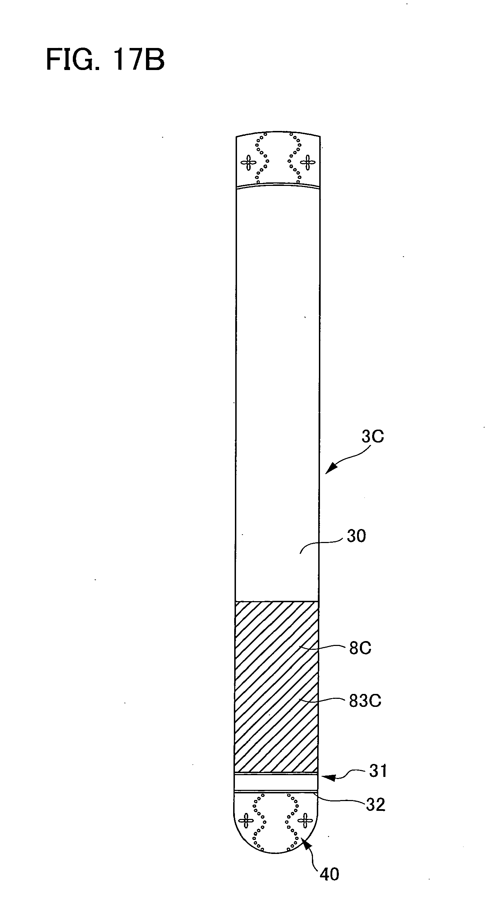

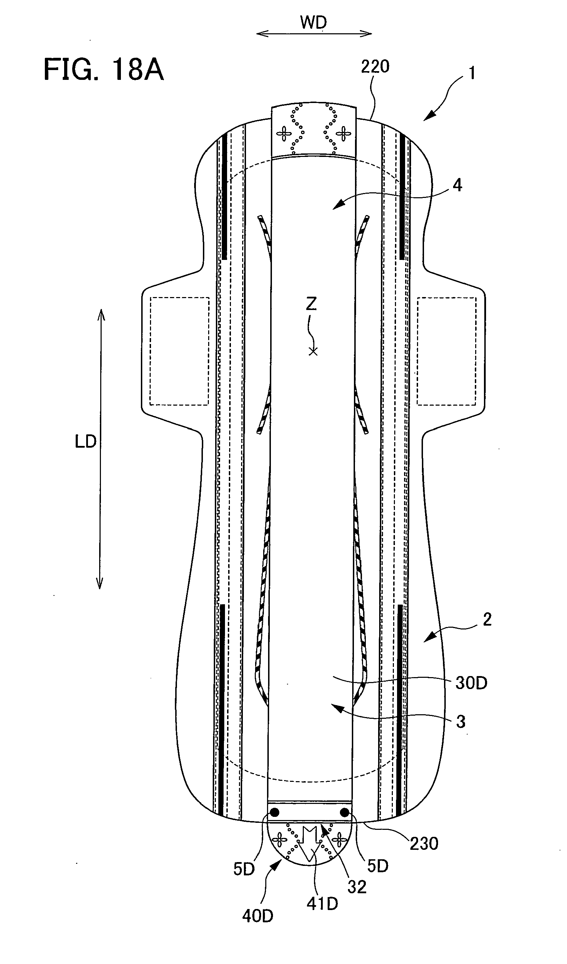

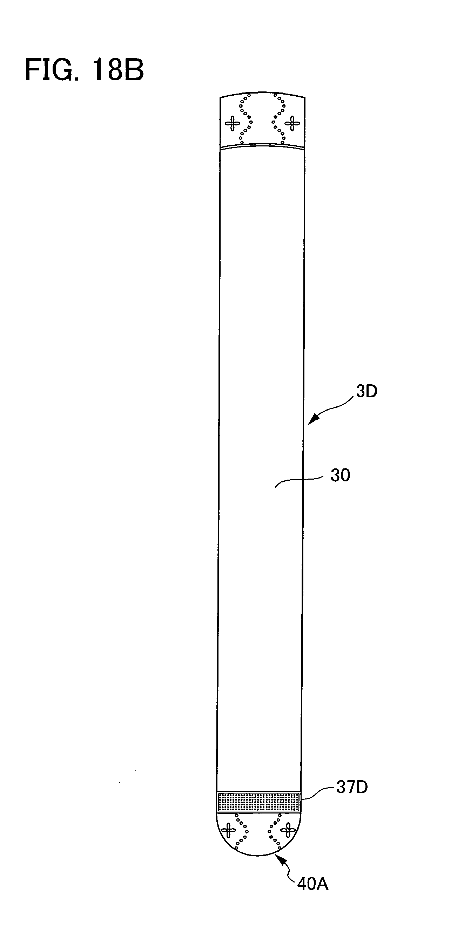

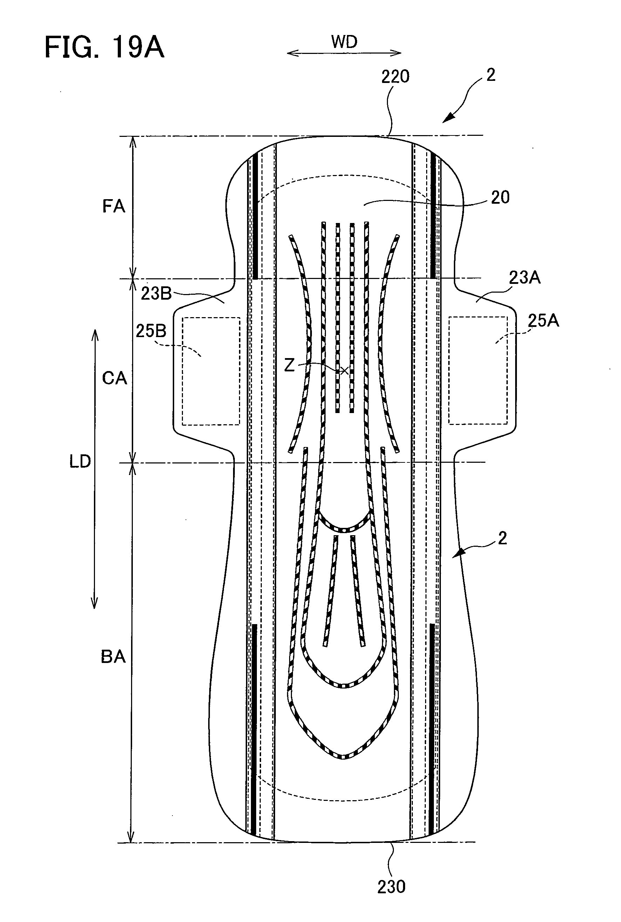

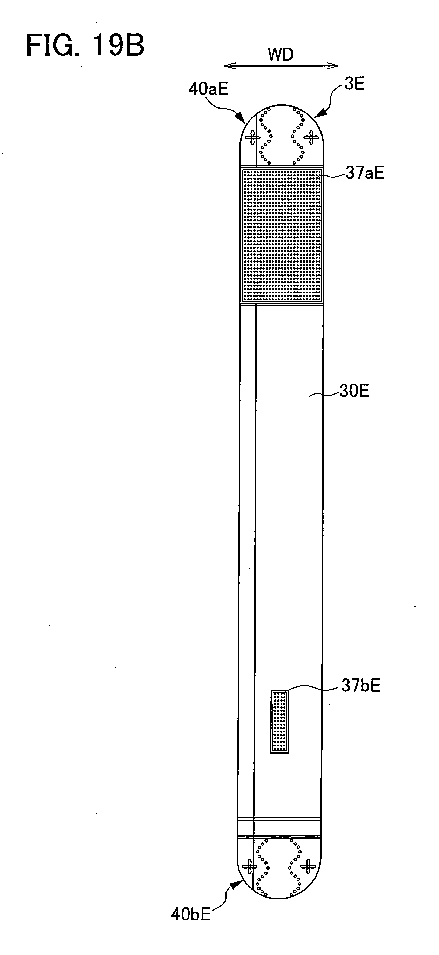

FIG. 15A is a plan view of a base absorbent core according to a second embodiment of the present invention. FIG. 15B is a back view of a top absorbent core according to the second embodiment of the present invention. FIG. 16A is a plan view of a base absorbent core according to a third embodiment of the present invention. FIG. 16B is a back view of a top absorbent core according to the third embodiment of the present invention. FIG. 17A is a plan view of a base absorbent core according to a fourth embodiment of the present invention. FIG. 17B is a back view of a top absorbent core according to the fourth embodiment of the present invention. FIG. 18A is a plan view of a base absorbent core according to a fifth embodiment of the present invention. FIG. 18B is a back view of a top absorbent core according to the fifth embodiment of the present invention. FIG. 19A is a back view of a top absorbent core according to a sixth embodiment of the present invention. FIG. 19B is a plan view of a base absorbent core according to the sixth embodiment of the present invention.

### 1. First Embodiment

An absorbent article 1 of the first embodiment of the present invention is described hereinafter with reference to FIGS. 1 to 12.

### 1.1. Overview

As shown in FIGS. 1 to 10, the absorbent article 1 according to the present embodiment is a substantially elongated absorbent article. The absorbent article 1 includes an elongated base absorbent core 2 and a top absorbent core 3 disposed on one surface of the base absorbent core 2 along a longitudinal direction LD of the base absorbent core 2. Regarding the top absorbent core 3, at least a part thereof in the longitudinal direction LD is fixed to the base absorbent core 2, and an end portion that is not fixed is spaced apart from the base absorbent core 2 having the part fixed as a starting point, being a free end 31 that can move independently.

The base absorbent core 2 is arranged on the garment side and absorbs a predetermined liquid such as menstrual blood that cannot be absorbed by the top absorbent core 3. In the base absorbent core 2, a strip-shaped central portion 20 is formed along the longitudinal direction LD nearly at the center in a width direction WD of the base absorbent core 2. Wings 23A and 23B are respectively formed so as to project outward in the width direction WD on both sides in the width direction WD of the absorbent article 1.

Here, the absorbent article 1 has a position Z as a first position with which the excretory part of a body is supposed to come into contact. The position Z is an intersection of a centerline Y extending along the longitudinal direction LD at the center in the width direction WD of the absorbent article 1 and a centerline B - B extending along the width direction WD at the center in the longitudinal direction LD of the wings 23A and 23B. In other words, the wings 23A and 23B are formed so as to satisfy the abovementioned positional relationship. In addition, in the base absorbent article 2, a region where the wings 23A and 23B are arranged, a region ahead of the region where the wings 23A and 23B are arranged, and a region behind the position where the wings 23A and 23B are arranged are taken as a central region CA, an anterior region FA, and a posterior region BA, respectively. The details thereof will be described later.

The top absorbent core 3 is arranged to be layered in a central portion 20 of the base absorbent core 2. The top absorbent core 3 absorbs a predetermined liquid such as menstrual blood, by being in direct contact with an excretory part and the like of a wearer's body during wearing. The top absorbent core 3 is fixed to the base absorbent core 2 by way of a fixing portion 4 provided in a part thereof in the longitudinal direction LD. An end portion on a side that is not fixed by the fixing portion 4 is made to be a free end 31. In a case where the top absorbent core 3 is fixed to the base absorbent core 2 at a position other than an end portion thereof, the free end 31 is an end being farther spaced apart from the fixing portion 4 in the longitudinal direction LD, for example.

On the free end 31 side of the top absorbent core 3, a handle portion 40 is disposed. When the absorbent article 1 is worn, the handle portion 40 is gripped and the free end 31 side of the top absorbent core 3 is spaced apart from the base absorbent core 2, with the fixing portion 4 as a starting point, thus disposing the top absorbent core 3 along a gluteal cleft, which is a groove in the vicinity of an excretory part of a wearer's body.

A temporary fixing portion 5 is formed on the top absorbent core 3. The temporary fixing portion 5 temporarily fixes the top absorbent core 3 to the base absorbent core 2, while allowing the top absorbent core 3 to be spaced apart therefrom with a predetermined force. Mobility of the top absorbent core 3 is limited in a state being temporarily fixed by way of the temporary fixing portion 5, and the mobility is not limited in a state of being released from temporary fixing. The top absorbent core 3 is spaced apart from the base absorbent core 2, with the fixing portion 4 as a starting point, in a state of being released from temporary fixing by the temporary fixing portion 5.

The top absorbent core 3 is disposed so as to be able to be spaced apart from the base absorbent core 2 from the fixing portion 4 as a starting point. In other words, the top absorbent core 3 can move (displace and deform) independently from the base absorbent core 2. As a result, the absorbent core can be continuously adhered to a wearer's body without being affected by movement of clothing, to which the base absorbent core 2 is attached. Individual components will be described hereinafter in detail. 1.2. Top Absorbent Core

As shown in FIGS. 1 to 8, the top absorbent core 3 is a substantially elongated absorbent core that is disposed on one surface of the base absorbent core 2, which is a skin contacting surface. More specifically, the top absorbent core 3 can be disposed along the central portion 20 of the base absorbent core 2, and includes a top absorbent portion 30 including an absorbent core 35, and a substantially plate-like handle portion 40 that is disposed in the vicinity of the free end 31. One end portion in the longitudinal direction LD of the top absorbent core 3 is fixed by way of the fixing portion 4. Additionally, the other end portion that is not fixed is the free end 31. An end portion, on the free end 31 side, of the top absorbent portion 30 is made to be a free end portion 32. Although the fixing portion 4 can be formed in an arbitrary position in the longitudinal direction LD of the top absorbent core 3, the fixing portion of the present embodiment is formed on one end side, which is located to the side of a front edge 220 when the top absorbent core 3 is disposed on the base absorbent core 2. Additionally, another end disposed to the side of a rear edge 230 is the free end 31.

As shown in FIG. 4, the free end 31 can be spaced apart from the base absorbent core 2. The top absorbent core 3 is configured so that the free end 31 can be spaced apart, with the fixing portion 4 as a starting point, while being disposed substantially in a center in the width direction WD of the base absorbent core 2. The free end 31 is a remote end portion from the fixing portion 4, and with a longer distance between the free end portion 32 and the fixing portion 4, a degree of freedom of the top absorbent portion 3 becomes greater.

As the free end 31 is formed in a position a predetermined distance apart from the fixing portion 4, a position of the top absorbent core 3 being worn can be appropriately adjusted by adjusting a position of the free end 31.

The top absorbent core 3 is disposed, for example, with portions extending from the front edge 220 of the base absorbent core 2 by 5 mm, and from the rear edge 230 thereof by 20 mm. The state extended 5 mm from the front edge 220 is provided in consideration of misalignment due to an error in manufacturing. In addition, by extending the top absorbent core 3 by 20 mm from the rear edge 230, gripping of the handle portion 40 can be facilitated for a case where the handle portion 40 is gripped in order to pull up the free end 31 of the top absorbent core 3. Furthermore, the handle portion 40 can be formed in the portions being extended.

As shown in FIGS. 3A, 3B, 3C, and 4, the top absorbent portion 30 includes a top layer 33 arranged on the skin contacting side, an absorbent core 35, and a back surface sheet 34 serving as a leakage-proof layer arranged on the base absorbent core 2 contacting side in the top absorbent portion 30. The top absorbent portion 30 is a principal member that absorbs a predetermined liquid excreted from the excretory opening.

A surface layer 33 is formed by performing perforation processing on a top sheet 331 and a second sheet 332, which are arranged to be layered. The surface layer 33 is formed by, together with forming a plurality of holes by the abovementioned perforation processing, integrating the top sheet 331 and the second sheet 332. The second sheet 332 is arranged so as to cover a surface, on the skin contacting side, of the absorbent core 35.

The top sheet 331 is arranged on the skin contacting side in the second sheet 332, and is arranged so as to wrap the whole of the second sheet 332, the absorbent core 355, described later, and the back surface sheet 34. The top sheet 331 forms an outermost surface of the top absorbent portion 30. The top sheet 331 is doubly arranged on the base absorbent core 2 contacting side in the top absorbent portion 30. It should be noted that the same member as the top sheet 27 in the base absorbent core 2, described later, can be used for the top sheet 331.

As shown in FIGS. 3A and 4, an end portion, on the side of the anterior region FA in the longitudinal direction LD, of the top absorbent portion 30 is in a state where only the top sheet 331 is triple-layered. The fixing portion 4 fixed to the base absorbent core 2 is arranged at the end portion on the side of the anterior region FA. It should be noted that it is preferable in a portion where the top sheet 331 is triple-layered, for layers to be bonded to one another with hot melt adhesives.

As shown in FIGS. 3C and 4, an end portion on the side of the posterior region BA in the longitudinal direction LD, of the top absorbent portion 30 is in a state where the top sheet 331 and the back surface sheet 34 are folded in three with the sheets layered and arranged to be triple-layered. Additionally, the handle portion 40, described later, is arranged at the end portion on the side of the posterior region BA. In addition, layers of each of the top sheet 331 and the back surface sheet 34 that are arranged to be layered are bonded to one another with hot melt adhesives at the end portion on the side of the posterior region BA.

The second sheet 332 is arranged on the skin contacting side in the top absorbent portion 30 so as to cover a top surface of the absorbent core 35, described later. In addition, the second sheet 332 is arranged to be layered between the top sheet 331 and the absorbent core 35, described later.

It is preferable for the second sheet 332 to be formed so as to be slightly larger than the absorbent core 35. In the present embodiment, the length in the longitudinal direction LD of the second sheet 332 is 300 mm, and the length thereof in the width direction WD is 45 mm.

The second sheet 332 according to the present embodiment is formed of an air-through non-woven fabric composed of a fiber having a core-sheath structure using polypropylene as a core and polyethylene as a sheath, for example, and having a fineness of 3.3 dtex and a length of 51 mm. Furthermore, the basis weight of the second sheet 332 is 20 g/m², for example. It is preferable that the second sheet 332 is formed such that the density thereof is higher than that of the top sheet 331. The second sheet 332 is higher in density than the top sheet 331, thereby allowing liquid migration from the top sheet 331 to be enhanced. In addition, a density gradient may be provided by not arranging the second sheet 332, but arranging the top sheet 331 one layer over the other.

Furthermore, as shown in FIGS. 3C and 4, the top absorbent portion 30 includes a back surface sheet 34 having liquid impermeability serving as a leakage-proof layer. The back surface sheet 34 is at least one portion, on the base absorbent core contacting side, of the absorbent core 35 and is arranged on the side of the rear edge 230.

It is preferable for the back surface sheet 34 to be arranged to a side surface of the absorbent core 35 from the base absorbent core contacting side and to not be arranged on the skin contacting side, as shown in FIG. 3C. This allows a predetermined liquid excreted in the top absorbent portion 30 to be absorbed in the absorbent core 35 when the liquid flows toward the rear edge 230. An SMS non-woven fabric having a basis weight of 24 g/m² and being composed of three layers, i.e., spun bond, meltblown, and spun bond layers formed of a film having liquid impermeability or a hydrophobic fiber, for example, can be used for the back surface sheet 34.

The absorbent core 35 mainly absorbs and holds the excreted predetermined liquid. The absorbent core 35 is formed of a pulverized pulp and a highly absorbent polymer. Here, it is preferable that the pulverized pulp is arranged such that the basis weight thereof partially differs in the top absorbent portion 30. More specifically, the basis weight of a region 353 where the fixing portion 4 is formed is 200 g/m², and the basis weight of regions 354, 355, and 356 from the fixing portion 4 to the free end portion 32 is 500 g/m², as shown in FIG. 5.

In addition, nondense portions 351 and 352 are formed in the posterior region BA in the top absorbent portion 30, as shown in FIG. 5. The nondense portion 351 is formed along the width direction WD on the side of the central region CA in the posterior region BA in the top absorbent portion 30, and the nondense portion 52 is formed along the width direction WD on the side of the rear edge 230 in the posterior region BA in the top absorbent portion 30.

The nondense portions 351 and 352 are regions where the weight of the pulverized pulp is lower than that in the other region. Additionally, the nondense portions 351 and 352 are fold starting points in a case where the absorbent article 1 is folded.

This inhibits, when the absorbent article 1 is folded when it is individually packed, for example, a wrinkle from appearing due to a difference in curvature between the inner side and the outer side of a fold.

Furthermore, a region 357 having a basis weight of 200 g/m² is formed in a vertically-long shape substantially at the center in the width direction WD of the top absorbent portion 30 in a region having a length of 80 mm directed toward the free end portion 32 from a position slightly closer to the free end portion 32 in relation to the position Z. The region 357 leads to deformation in the top absorbent core 3 in a worn state.

The absorbent core 35 is formed such that the length thereof in the longitudinal direction LD is less than the length in the longitudinal direction LD of the top sheet 331. That is, the absorbent core 35 is not arranged at both ends in the longitudinal direction LD of the top absorbent portion 30, as described above.

It is desirable for the length in the longitudinal direction LD of the top absorbent core 3 to be, for example, 200 mm to 500 mm, and preferably 230 mm to 450 mm. An example of the length in the longitudinal direction LD of the top absorbent core 3 in the first embodiment is 335 mm. In the present embodiment, an example of the length in the longitudinal direction LD of the top absorbent portion 30 is 280 mm.

In addition, in the present embodiment, the length in the width direction WD of the top absorbent portion 30 is less than the length in the width direction WD of the base absorbent core 2. Furthermore, it is preferable for the top absorbent portion 30 to have a length such that it can come into contact with the base absorbent core 2 in the longitudinal direction LD along the gluteal cleft.

More specifically, it is desirable for the length in the width direction WD of the top absorbent portion 30 to be 15 mm to 50 mm, and preferably 20 mm to 40 mm. In a case where the width dimension of the top absorbent portion 30 is less than 15 mm, the width is not sufficient for a napkin to maintain contact with the vaginal opening. Therefore, a clearance between the wearer's body and the napkin is liable to occur so that menstrual blood easily leaks. The width dimension of the top absorbent portion 30 according to the first embodiment can be exemplified, for example, as being made to 40 mm.

In addition, it is preferable for the top absorbent portion 30 to have a substantially equal width in the longitudinal direction LD. Furthermore, it is preferable for the cross-sectional shape in the width direction WD of the top absorbent portion 30 to be in a state where the skin contacting side and the base absorbent core 2 contacting side are at least substantially parallel to each other.

### 1.3. Property of Top Absorbent Core

### 1.3.1 Flexural Rigidity

Due to the top absorbent core 3 being used to directly contact the wearer's body, flexural rigidity thereof may lead to an unpleasant sensation. For example, as shown in FIG. 12, although the top absorbent core 3 deforms in a worn state into a predetermined curve toward the surface of the wearer's body side, in this case, when there exist a significant rigidity, draping ability becomes poor as well as becoming difficult to fill gaps between the top absorbent core 3 and the wearer's body at grooves close to the body's excretory part and the like. In other words, in a case where the flexural rigidity is greater than a predetermined value, the top absorbent core 3 cannot follow appropriately the wearer's body shapes and menstrual blood and the like may leak from the gaps generated between the wearer's body. That is, it is preferred for the flexural rigidity of the top absorbent portion 30 toward the upper side to be lower than a predetermined value.

In the present embodiment, for example, the flexural rigidity of the top absorbent core 3 opposite to the side of the base absorbent core is 0.05 to 1.7 N, preferably 0.1 to 1.5 N, and more preferably 0.2 to 1.2 N. When the flexural rigidity is lower than 0.05 N, operability may be inferior during attaching since the top absorbent core 3 is excessively soft. Furthermore, when the flexural rigidity is greater than 1.7 N, familiality with the wearer's body may be deteriorated.

Furthermore, it is preferable for the flexural rigidity at the central region, from the position Z shown in FIG. 11 or 12 to the position of the top absorbent core 3 spaced apart 100 mm toward the side of the free edge 31, to be 0.1 to 1.5 N, preferably 0.2 to 1.0 N. It is Preferable for the central region to have a moderate softness since grooves of the body are particularly deep.

Preferably, the difference between the flexural rigidity at a predetermined position of the top absorbent core 3 and the flexural rigidity at another positions spaced apart from the predetermined position in the longitudinal direction LD is no greater than 1.2 N, preferably 1.0 N. For example, when the top absorbent core 3 has a site where the flexural rigidity is considerably different between adjacent regions in the longitudinal direction LD, large deformation occurs at the site, and the familiality with the wearer's body may be impaired in the top absorbent core 3. Furthermore, wastes such as menstrual blood may leak from the largely deformable site (where familiality with the wearer's body is poor). It is, therefore, preferred that the difference between the flexural rigidity at a predetermined position of the top absorbent core 3 and the flexural rigidity at the other positions spaced apart from the predetermined position in the longitudinal direction LD is within the above described range. For example, any bending positions of the bending positions A to D in FIG. 11 or 12 represent the difference of flexural rigidity of no greater than 1.2 N at the adjacent bending positions of A to D.

The value of flexural rigidity of the top absorbent core 3 is 0.1 to 2 times, preferably 0.2 to 1.5 times, and more preferably 0.3 to 1.2 times of the value of flexural rigidity of the base absorbent core 2 to the side of the top absorbent core 3. For example, when underwear is pulled up in a state where the base absorbent core 2 is attached to inside of the underwear or the like, the top absorbent core 3 and the base absorbent core 2 are curved in an overlapped state fitting along the underwear shape. Therefore, when the flexural rigidity of the top absorbent core 3 is at least two times the flexural rigidity of the base absorbent core 2, it may be difficult to curve the top absorbent core 3 and the base absorbent core 2 in combination. Consequently, the top absorbent core 3 and the base absorbent core 2 may space apart from each other prior to pulling up the underwear to contact the wearer's body. In this case, the top absorbent core 3 may not be disposed appropriately to a predetermined position (e.g. excretory part) due to dislocation upon pulling up. When the value of flexural rigidity of the top absorbent core 3 is in the range on the basis of the value of flexural rigidity of the base absorbent core 2 to the side of the top absorbent core 3, the spacing between the top absorbent core 3 and the base absorbent core 2 can be regulated. It is preferred since the top absorbent core 3 and the base absorbent core 2 can be curved in combination, for example.

Here, the flexural rigidity of the top absorbent core 3 and the base absorbent core 2 of the present invention can be measured by the measuring method described below. Measurement of Flexural rigidity

A digital force gage "FGC-xB series", article name, model FGC-2B manufactured by Nidec-Shimpo Co. was used for measuring the flexural rigidity.

The measuring procedures are as follows.
(1) A hot melt adhesive, coated on the dislocation preventing portion 26 of the base absorbent core 2 of a measuring object, is made to be non-adherent using Siccarol, and then wings 23A and 23B and gather are cut. The wings 23A and 23B are cut at the feet of the main body and the wings 23A and 23B. The gather is made to be cut at a plurality of sites thereof in the longitudinal direction LD so as to be free of stress by way of cutting the stretchable rubber. In the present embodiment, the top absorbent core 3 is measured without the processing described above.
(2) The measuring object is cut so that the length is 50 mm from a measuring site to the side to contact with a terminal of the digital force gauge.
(3) The measuring object is put on a table (back side facing upward) so as to extend 50 mm therefrom. At this time, measuring site (place to be bent) of the measuring object is matched with the edge of the clog table described above. That is, the measuring sample is set to extend 50 mm from the table edge.
(4) At this time, when the measuring object bends downward by its own weight, a filter paper is laid on the downside of the measuring object to prevent the bending by its own weight.
(5) A clog of 2 kg is put on to match the edge thereof with the measuring site of the measuring object.
(6) The digital force gage is driven downward vertically at a rate of 200 mm/min to the position of 5 mm from the table edge.
(7) The maximum load at lowering 5 mm is recorded. When filter paper is used, the flexural rigidity is determined by subtracting the flexural rigidity of the filter paper from the measured value. 1.3.2. Compression Hardness

Furthermore, since the top absorbent portion 30 directly contacts excretory areas of the wearer's body, it is desirable to have a compression hardness within a predetermined range at the contacting surface side.

For example, the compression hardness (Linearity of Compression) at the surface of the top absorbent core 3 opposite to the side of the base absorbent core 2 is 0 to 0.8 (-), preferably 0.4 to 0.75 (-) or less, and further 0.70 (-) or less. When the compression hardness (LC) is 0.4 (-) or less, change in the thickness of the top absorbent core 3 relative to pressure change becomes large, for example, and absorbability may be impaired due to higher density of the top absorbent core 3. When compression hardness (LC) is above 0.8 (-), for example, a rigid feeling may be given to the user, thereby decreasing familiality with the wearer's body, for example. It is, therefore, preferred for the compression hardness (LC) at the surface of the top absorbent core 3 opposite to the side of the base absorbent core 2 to be within the range. Furthermore, the compression hardness (LC) at the surface of the top absorbent core 3 opposite to the side of the base absorbent core 2 is the compression hardness (LC) from the surface of the top absorbent core 3 at the wearer's body side (skin side) toward the side of the base absorbent core 2.

In addition, the compression hardness (LC) at the central region is 0.3 to 0.75 (-), and preferably 0.4 to 0.7 (-). Since the central region is a region that contacts with body grooves and the like so as to fit thereto in particular, due to those having compressive softness being likely to contact thereto at groove depth, therefore, it is preferred that the compression hardness (LC) at the central region is within the range.

The value of the compression hardness (LC) at the surface of the top absorbent core 3 opposite to the side of the base absorbent core 2 is 0.1 to 2 times, preferably 0.2 to 1.5 times, and more preferably 0.3 to 1.2 times on the basis of the value of the compression hardness (LC) of the base absorbent core 2. For example, in a case where the top absorbent core 3 is used while not spacing apart from the base absorbent core 2 (top absorbent core 3 does not contact along with fitting body grooves), and in a case where the value of the compression hardness (LC) of the top absorbent portion 30 is significantly different from the value of the compression hardness (LC) of the base absorbent core 2, and in a case where the value of the compression hardness (LC) of the top absorbent core 3 is very large, the top absorbent core 3 may be embedded in the base absorbent core 2 making it difficult to contact the top absorbent core 3 properly with the wearer's body. 1.3.3. Compression Resilience Ratio

Compression resilience ratio (RC) of the top absorbent core 3 at the surface opposite to the side of the base absorbent core 2 is at least 30%, and preferably at least 35%. When the compression resilience ratio (RC) is at least 30%, for example, it is preferred for maintaining easily a so-called fit state, since even when the top absorbent core 3 enters into body grooves and is compressed by pressure, the shape is likely to recover. Here, the compression resilience ratio (RC) of the top absorbent core 3 at the surface opposite to the side of the base absorbent core 2 is the compression resilience ratio (RC) when the top absorbent core 3 is compressed from the surface of the wearer's body side (skin side) toward the side of the base absorbent core 2.

The compression resilience ratio (RC) at the central region of a region from the position, where the top absorbent core 3 contacts the excretory part, to the position of the top absorbent core 3 spaced apart 100 mm to the side of the free edge 31 is at least 35%, and preferably at least 40%. Since the central region is a region that contacts with body grooves and the like so as to fit thereto in particular, even when compressed by pressure, a so-called fit state tends to be maintained since the shape is easily recovered. Preferably, the compression resilience ratio (RC) at the central region is within the range described above. Measurement of Compression Hardness and Compression Resilience

Compression hardness (LC) and compression resilience (RC) in the present invention can be measured by the measuring methods described below.

KES compression tester (article name, model KES-G5-50) manufactured by Kato Tech Co. was used to measure the compression hardness and the compression resilience.

The measuring conditions were speed: 0.1 cm/sec, compression area: 2 cm², sensitivity: 2 (force meter 200 g/10v), compressive load: 50 gf/cm², and a measuring object is compressed and then LC (compression hardness) and RC (compression resilience) were calculated from a correlation chart between pressure and deformation volume. The compression hardness is evaluated to be harder as the value of the compression hardness (LC) approaches 1. The compression resilience (RC) is evaluated to be more recoverable as the value approaches 100%.

### 1.3.4. Entering Property

The top absorbent portion 30 to contact the wearer's body in the top absorbent core 3 is provided with a predetermined fold starting point element in the top absorbent portion 30 along the longitudinal direction LD substantially in a center of the top absorbent portion 30 in the width direction WD. Specific examples of the fold starting point element are described later.

The predetermined fold starting point element can be disposed, as shown in FIG. 5, so that the basis weight of the pulverized pulp used as an absorbent core is different locally in the top absorbent portion 30, for example. More specifically, the basis weight is 200 g/m² at the region 353 where the fixed portion 4 is formed, and the basis weight is 500 g/m² at the regions 354, 355, and 356 from the fixed portion 4 to the free end portion 32.

Furthermore, as shown in FIG. 5, nondense portions 351, 352 are formed at the rear region BA of the top absorbent portion 30. The nondense portion 351 is formed at the side of the central region CA of the rear region BA of the top absorbent portion 30 along the width direction WD, and the nondense portion 352 is formed at the side of the rear edge 230 of the rear region BA of the top absorbent portion 30 along the width direction WD.

The nondense portions 351, 352 are a region where the basis weight of pulverized pulp is smaller than those of other regions. Additionally, the nondense portions 351 and 352 act as a fold starting point when the absorbent article 1 is folded. Here, the term "near the free end" refers to a region from the outer edge of the handle portion 40 to one-fourth of total length of the top absorbent core 3 containing the handle portion 40.

Consequently, when the absorbent article 1 is folded for individual packaging and the like, wrinkles are prevented from generating due to curvature differences of inner side and outer side of folding.

Moreover, a region 357 having an basis weight of 200 g/m² is formed longitudinally substantially in a center of the top absorbent portion 30 in the width direction WD at the region 357 of 80 mm long from the position slightly shifted to the side of the free end portion 32 of the position Z. The region 357 induces a deformation of the top absorbent core 3 in a worn state.

Preferably, the region 357 is formed at least between the position Z where the body excretory part are intended to contact and the nondense portion 352 in the longitudinal direction LD. The vaginal opening as the first excretory area and the anus as the second excretion area are intended to contact between the position Z and the nondense portion 352 in the longitudinal direction LD. More specifically, the region 357 is formed substantially in a center of the top absorbent portion 30 in the width direction and from the position slightly shifted from the position Z toward the side of the rear edge 230 to the position of length 80 mm in length toward the rear edge 230. In this way, the fold starting point element is formed by forming a difference of basis weight in the top absorbent portion 30.

The basis weight of pulverized pulp, in the present embodiment, is exemplified by a case in which it is 200 g/m² at the region 357 of 10 mm made so that the center is the middle of the top absorbent portion 30 in the width direction WD and 500 g/m² at the regions 354 and 355 outside therefrom in the width direction WD, for example. By the basis weight being lower at the central region of the top absorbent portion 30 in the width direction WD, the central region becomes a fold starting point element, and thus the top absorbent portion 30 easily enters into the gluteal cleft while projecting to the surface side in the thickness direction as shown in FIG. 13.

It should be noted that an absorbent fiber with a fiber length longer than that of pulp may be disposed at the skin contacting side of pulverized pulp in order to improve skin contact when the top absorbent portion 30 contacts the wearer's body. The construction of the absorbent fiber with a fiber length longer than that of pulp is exemplified by those formed of 60% by mass to 90% by mass of a rayon with 3.3 dtex and fiber length 51 mm and 10% by mass to 40% by mass of a cotton and set the basis weight to 50 g/m² to 500 g/m². By mixing to be layered the absorbent fiber with a fiber length longer than that of pulp formed in this way is mixed and laminated and disposing at the skin contacting side, skin contact can be improved when the top absorbent portion 30 contacts the wearer's body.

Furthermore, another example of the fold starting point element formed at the top absorbent portion 30 along the longitudinal direction LD is exemplified by a slit 61, as shown in FIG. 14A for example, that is formed to be continuous or intermittent substantially at the center of the top absorbent portion 30 in the width direction WD along the longitudinal direction LD of the top absorbent portion 30.

In addition, another example is exemplified by a space portion 63, as shown in FIG. 14B for example, that is formed to be continuous or intermittent substantially in a center of the top absorbent portion 30 in the width direction WD along the longitudinal direction LD of the top absorbent portion 30.

The absorbent core 64 of the top absorbent portion 30 in the aforementioned case is formed of an air raid pulp that is made to be a composite of 60:10:30 of a pulp, an absorbent polymer, and a composite fiber having a polypropylene core and a polyethylene sheath. The basis weight of the air raid pulp is exemplified by 200 g/m².

It should be noted that the air raid pulp having a tendency to have a hard feel of skin contact when contacting with skin, therefore, a pulverized pulp or an absorbent fiber with a fiber length longer than that of pulp can be disposed in the air raid pulp at the body contacting side. In a case where a pulverized pulp or an absorbent fiber with a fiber length longer than that of pulp is disposed, since the top absorbent portion 30 is thickened in the thickness direction, therefore, it is preferred that a nondense portion of 1 mm to 5 mm of a space portion with a predetermined width is formed rather than the slit 61 described above.

Furthermore, the fold starting point element is exemplified by a region having a different fiber basis weight at outside of the top absorbent portion 30 in the width direction WD that is formed to be continuous or intermittent substantially at the center of the top absorbent portion 30 in the width direction WD along the longitudinal direction LD, for example.

For example, first, a case can be exemplified in which the basis weight is higher at the central region of the side absorbent portion 30 in the width direction and the basis weight is lower at the outer region in the width direction WD.

More specifically, as shown in FIG. 14C, the case can be exemplified in which the top absorbent portion 30 has a central region 65 formed substantially at the center in the width direction WD and an outer region 66 formed outside of the central region 65 in the width direction WD.

Additionally, in this case, when the difference between the basis weight at the central region 65 and the basis weight at the outer region 66 is within a predetermined range, the top absorbent portion 30 has appropriately a bendability and an absorbability.

For example, a case can be exemplified in which the difference in basis weight between the central region 65 and the outer region 66 of the pad side absorbent portion in the width direction is 50 g/m² to 1000 g/m², preferably 100 g/m² to 800 g/m², and particularly preferably 150 g/m² to 600 g/m².

Furthermore, the fold starting point element is exemplified by a region having a different fiber density at outside of the top absorbent portion 30 in the width direction WD that is formed to be continuous or intermittent substantially at the center of the top absorbent portion 30 to configure the pad side absorbent layer in the width direction WD along the longitudinal direction LD, for example.

For example, first, a case can be exemplified in which the density is higher at the central region 65 of the side absorbent portion 30 in the width direction WD and the density is lower at the outer region 66 in the width direction WD. Next, a case can be exemplified in which the density is lower at the central region of the side absorbent portion 30 in the width direction WD and the density is higher at the outer region in the width direction WD.

More specifically, as shown in FIG. 14C, the case can be exemplified in which the top absorbent portion 30 has a central region 65 formed at middle in the width direction WD and an outer region 66 formed outside of the central region 65 in the width direction WD.

Additionally, in this case, when the difference between the density at the central region 65 and the density at the outer region 66 is within a predetermined range, the top absorbent portion 30 has appropriately a bendability and an absorbability.

For example, a case can be exemplified in which the difference in density between the central region 65 and the outer region 66 of the top absorbent portion 30 in the width direction WD is 0.005 g/cm³ to 0.5 g/cm³, preferably 0.01 g/cm³ to 0.3 g/cm³, particularly preferably 0.015 g/cm³ to 0.2 g/m³.

More specifically, as shown in FIG. 14C, the case can be exemplified in which the top absorbent portion 30 has a central region 65 formed at the middle in the width direction WD and an outer region 66 formed outside of the central region 65 in the width direction WD.

Furthermore, the fold starting point element is exemplified by a predetermined folding line that is formed to be continuous or intermittent by previously doing a fold processing at substantially the center of the top absorbent portion 30 to configure the pad side absorbent layer in the width direction WD along the longitudinal direction LD, for example.

As shown in FIG. 13, the central portion in the width direction WD is made to project to the surface side of the top absorbent portion 30 to form a predetermined folding line by way of a predetermined apparatus and the like. Therefore, the absorbent article 1 can deform so as to fold along the broken line in actual use.

Furthermore, the fold starting point element is exemplified by a core portion that is disposed substantially in a center of the top absorbent portion 30 to configure the pad side absorbent layer in the width direction WD along the longitudinal direction LD, for example.

The core portion is exemplified, as shown in FIG. 14D, by a predetermined compressed portion 67 that is formed to be continuous or intermittent substantially at the center of the top absorbent portion 30 in the width direction WD along the longitudinal direction LD.

When the absorbent core 35 of the top absorbent portion 30 includes a pulverized pulp as a main material, the absorbent core 68 is formed by wrapping a blend body of the pulverized pulp and an absorbent polymer with a tissue having a basis weight of 15 g/m². It should be noted that the absorbent polymer is exemplified by that having a basis weight at the top absorbent portion 30 which is substantially uniformly 15 g/m², for example. The basis weight of the pulverized pulp is exemplified in the present embodiment by 500 g/m², for example.

Additionally, a pin-shaped emboss can be formed at predetermined intervals at the substantially central region of the top absorbent portion 30 in the width direction WD. Consequently, a core portion is formed at the substantially central region of the top absorbent portion 30 in the width direction WD and the central region comes to a fold starting point, thereby the top absorbent portion 30 is likely to enter into the gluteal cleft while deforming into a convex-shape toward the surface side as shown in FIG. 13.

It should be noted that it is preferred for the embossing process to be performed from the face of the top absorbent portion 30 at the side of the base absorbent core 2.

Furthermore, an absorbent fiber with a fiber length longer than that of pulp may be disposed at the skin contacting side of the pulverized pulp. The configuration of the absorbent fiber with a fiber length longer than that of pulp is exemplified by those formed of 60% by mass to 90% by mass of a rayon of 3.3 dtex and a fiber length of 51 mm and 10% by mass to 40% by mass of a cotton and set the basis weight set to 50 g/m² to 500 g/m². By mixing and laminating the absorbent fiber with a fiber length longer than that of pulp formed in this way then disposing at the skin contacting side, skin contact can be improved when the top absorbent portion 30 contacts the wearer's body.

Furthermore, the core portion of a fold starting point element can be similarly exemplified, as shown in FIG. 28E, by a tubular absorbent core 69 that is formed by way of winding a predetermined non-woven fabric and the like to an outer circumference of a predetermined fiber, which forms the absorbent core 139 of the top absorbent portion 30, thereby forming a tubular shape, for example.

Since the rigidity of the tubular absorbent core 69 is higher (harder) than that of the outer region 70 in the width direction where the fiber configuring the absorbent core 139 is disposed, therefore, the tubular absorbent core 69 comes to a fold starting point to deform as shown in FIG. 13. That is, the substantial middle of the top absorbent portion 30 in the width direction WD deforms so as to project toward the skin contacting side (arrow F) opposite to the side of the base absorbent core 2 (not shown). Consequently, it can contact so as to enter into a predetermined groove of the wearer's body.

Furthermore, another example of the fold starting point element can be exemplified by a portion where a hot melt adhesive is linearly coated at a site where the top sheet 331 is doubled at the side of the top absorbent portion 30 to contact the base absorbent portion. Since the portion, where the top sheet 331 is doubled and also the hot melt adhesive is coated, has a higher rigidity, therefore, the portion becomes to a fold starting point.

### 1.4. Fixing portion

As shown in FIGS. 1 and 4, the fixing portion 4 is disposed in an arbitrary position on the top absorbent core 3, and fixes the top absorbent core 3 to the base absorbent core 2. More specifically, a predetermined region in the longitudinal direction LD of the top absorbent core 3 is fixed to the base absorbent core 2 by way of the fixing portion 4. An end portion of the top absorbent core 3 that is not fixed by the fixing portion 4 is made to be a free end.

As shown in FIGS. 1 and 7, the fixing portion 4 is formed in a position to the side of the front edge 220 of the top absorbent core 3 in a state of being placed on the base absorbent core 2. More specifically, the fixing portion 4 is formed in a position in an end portion of the top absorbent portion 30 to the side of the front edge 220, the portion corresponding to a zone from a region formed only of the top sheet 331 to a region 353 where the weight of the absorbent core 35 is 200 g/m².

The fixing portion 4 is composed of a pressure-bonding portion 46 and a joining portion 47. The joining portion 47 is formed by applying a hot melt adhesive in the region formed only of the top sheet 331 and in the region in which the absorbent core 35 is disposed in the top absorbent core 3, at substantially regular intervals along the longitudinal direction LD, and placing and bonding the top absorbent core 3 substantially in a center in the width direction WD of a front region FA of the base absorbent core 2. The hot melt adhesive is preferably applied so as to face a portion in the front region FA of the base absorbent core 2, where the compressed groove 22 is not disposed. In addition, the hot melt adhesive is applied in a region from a predetermined position between a position Z and the front edge 230, to the front edge 230. In other words, the fixing portion 4 is formed in a position closer to the front edge 220 than the position Z is. It should be noted that the fixing portion 4 can be formed by integrating the top absorbent core 3 and the base absorbent core 2 by a compressed groove, instead of the hot melt adhesive. In such a case, a region where the base absorbent core 2 and the top absorbent core 3 are fixed by the compressed groove is the fixing portion 4.

The pressure-bonded portion 46 is formed in a portion on the base absorbent core 2 to the side of the front edge 220, where the absorbent core 28 is not disposed. The pressure-bonded portion 46 is formed in the region on the base absorbent core 2, along with the region formed only of the top sheet 331 of the top absorbent portion 30.

The fixing portion 4 is formed by joining by a pressure bonding process for integrating the surface layer 33 and the absorbent core 35 of the top absorbent core 3 with the base absorbent core 2.

### 1.5. Temporary Fixing Portion

As shown in FIG. 1, a temporary fixing portion 5 is formed on the top absorbent core 3. The temporary fixing portion 5 temporarily fixes the top absorbent core 3 to the base absorbent core 2, while allowing the top absorbent core 3 to be spaced apart therefrom with a predetermined force. Mobility of the top absorbent core 3 is limited in a state being temporarily fixed by means of the temporary fixing portion 5, and the mobility is not limited in a state of being released from temporary fixing. The top absorbent core 3 is spaced apart from the base absorbent core 2, with the fixing portion 4 as a starting point, in a state of being released from temporary fixing by the temporary fixing portion 5. The temporary fixing portion 5 is formed on the top absorbent core 3, between the fixing portion 4 and the free end portion 32. The temporary fixing portion 5 is formed on the base absorbent core 2 in the vicinity of the rear edge 230 of the central portion 20.

More specifically, the temporary fixing portion 5 is formed on the top absorbent core 3, in the vicinity of the free end 31. The temporary fixing portion 5 is, more specifically, formed on both sides in the width direction WD of the free end portion 32.

The temporary fixing portion 5 is formed in a region in the top absorbent core 3 formed only of the top sheet 331 and a back surface sheet 34. Furthermore, the temporary fixing portion 5 is formed in the vicinity of the handle portion 40 of the top absorbent core 3. A force applied outwardly in the longitudinal direction LD of the handle portion 40, or applied toward an upper face in FIG. 1, is directly transferred to the temporary fixing portion 5. In other words, in a case where the handle portion 40 is displaced outwardly in the longitudinal direction LD or toward the upper face in FIG. 1, temporary fixing by the temporary fixing portion 5 is released.

As described above, the temporary fixing portion 5 is formed in a circular shape on both sides in the width direction WD of the free end portion 32, in the vicinity of the handle portion 40 on the top absorbent core 3. The top absorbent core 3 is fixed at points by two of the temporary fixing portions 5 and 5.

The temporary fixing portion 5 is formed on the base absorbent core 2 to the side of the rear edge 230 of the base absorbent core 2. The temporary fixing portion 5 is formed in a region in the base absorbent core 2 formed only of the top sheet 27 and a back surface sheet 29.

The temporary fixing portion 5 is formed by arranging the top absorbent core 3 to be layered on the base absorbent core 2 and performing an embossing process (a pressure bonding process) from above the top absorbent core 3.

More specifically, the temporary fixing portion 5 is formed by processing the free end portion 32 formed only of the top sheet 331 and the back surface sheet 34 of the top absorbent core 3, and a region in the base absorbent core 2 where the top sheet 27 and the back surface sheet 29 are arranged to be layered, by performing pressure bonding process with an embossing member having concave and convex portions on the surface thereof. As a result, the temporary fixing portion 5, which is an embossed portion, fixes the top absorbent core 3 and the base absorbent core 2 with mild thermal fusion bonding.

### 1.6. Handle Portion

As shown in FIGS. 1 and 4, the handle portion 40 is disposed to the side of the free end 31 in the longitudinal direction LD of the top absorbent portion 30. The handle portion 40 is a portion pinched and pulled by a user in order to adjust a position of the top absorbent core 3, in a process of putting on the absorbent article 1. The handle portion 40 is formed to extend the most outwardly in the longitudinal direction LD in a central portion in the width direction WD.

An outer edge portion of the handle portion 40 has a curved shape. More specifically, the outer edge portion of the handle portion 40 can be formed in a substantially semicircular shape having an apex at an intersection of an extended line of a substantial center in the width direction WD of the top absorbent core 3 and an outer edge.

The handle portion 40 is disposed to the side of the free end 31 and formed in a region to the side of the free end 31 of the top absorbent portion 30, where the top absorbent portion 30 does not have the absorbent core 35 and where only the top sheet 331 and the back surface sheet 34 are extended.

The handle portion 40 is formed by increasing the rigidity of the free end portion 32 of the top absorbent portion 30. More specifically, the handle portion 40 is formed by performing an embossing process to the free end portion 32 of the top absorbent portion 30, which is a region where only the top sheet 331 and the back surface sheet 34 are extended, in order to increase the rigidity thereof. On the handle portion 40, a small, circular shaped embossed portion is formed in an hourglass shape, and a flower-shaped embossed portion is formed on a side portion thereof. The hourglass-shaped embossed portion acts as a guide element for a position to place a finger when a user grips the handle portion 40. An elaborately designed handle portion 40 can be provided by devising a shape of the embossed portion. In other words, an elaborately designed handle portion 40 can be provided by the embossing process to increase rigidity. In other words, a predetermined function and a design can be provided in a process for imparting rigidity.

For the design (the guide element), an arbitrary shape can be given by changing a shape of the embossed portion. For example, an indication for prompting a user to pinch and pull the handle portion 40 to a predetermined direction in the longitudinal direction LD can be provided. More specifically, an outward pointing arrow in the longitudinal direction LD that prompts a user to pull the handle portion 40 to the longitudinal direction LD, a dot indicating a position to pinch, and a predetermined combination of colors can be used.

In addition, concave and convex portions are formed on a surface of the handle portion 40 by performing the embossing process. The concave and convex portions can be an indicator for finding the handle portion 40 by touch, which is disposed in a position invisible from a user, in a wearing process of the absorbent article.

As described above, the temporary fixing portions 5 and 5 are formed in the vicinity of the handle portion 40. Since the temporary fixing portions 5 and 5 are formed in positions that follow the displacement of the handle portion 40, the temporary fixing by the temporary fixing portions 5 and 5 is released only by displacing the handle portion 40 by a predetermined distance. More specifically, by displacing the handle portion 40 for displacing a portion in the vicinity of the free end 31 to another position, the temporary fixing by the temporary fixing portions 5 and 5 is released.

In addition, the handle portion 40 is disposed so as to project outwardly from the rear edge 230 of the base absorbent core 2. A portion of the handle portion 40 projecting outwardly from the rear edge 230 appropriately functions as a grip portion. However, an end portion of the handle portion 40, to the side of the rear edge 230, is not necessarily required to project from the base absorbent core 2 and may be disposed inside the rear edge 230 of the base absorbent core 2.

An outer edge of the handle portion 40 is preferable disposed so as to project from the rear edge 230 of the base absorbent core 2. More specifically, a range of 100 mm outward to 50 mm inward, preferably 60 mm outward to 30 mm inward, and more preferably 30 mm outward to 20 mm inward, from the rear edge 230 of the base absorbent core 2, can be exemplified as a starting point. By disposing the outer edge portion of the handle portion 40 so as to project from the rear edge 230 of the base absorbent core 2, it becomes easier for a user to grip. For example, the handle portion 40 can be found more easily, when a user tries to locate the handle portion 40 on her back side when wearing of the absorbent article. In addition, a portion of the handle portion 40 projecting outwardly from the rear edge 230 appropriately functions as a grip portion.

Flexural rigidity (B) of the handle portion 40 is 0.1 to 1.2 (10⁻⁴N·m²/m), preferably 0.2 to 1 (10⁻⁴N·m²/m), and more preferably 0.3 to 0.8 (10⁻⁴N·m²/m). In a case where the flexural rigidity (B) of the handle portion 40 is lower than 0.1 (10⁻⁴N·m²/m), for example, a predetermined operation may be difficult such as gripping the handle portion 40 to position the top absorbent core on a predetermined portion of a wearer's body. On the contrary, in a case where the flexural rigidity (B) of the handle portion 40 is greater than 10, the handle portion may give an unpleasant sensation to a wearer during wearing. Thus, the flexural rigidity (B) of the handle portion 40 is preferably in the abovementioned range.

Flexure recovery (2HB) of the handle portion 40 is no greater than 10 (10⁻²N·m/m), preferably no greater than 7, and more preferably no greater than 3 (10⁻²N·m/m). In a case where the flexure recovery (2HB) of the handle portion 40 is greater than 10 (10⁻²N·m/m), creases may easily be formed when the handle portion 40 is folded back, and may give an unpleasant sensation to a wearer. Thus the flexure recovery (2HB) of the handle portion 40 is preferably in the abovementioned range.

The thickness of the handle portion 40 being 0.5 to 4 mm, and preferably 0.7 to 3.5 mm is preferred. In a case where the thickness of the handle portion 40 is less than 0.5 mm, a user may worry that the handle portion may be torn when the user grips and pulls the handle portion 40. On the contrary, in a case where the thickness of the handle portion 40 is greater than 5 mm, the handle portion 40 may give a user a sensation of a foreign-object during use.

### Measuring Method

Measuring method of the flexural rigidity (B) and the flexural recovery (hysteresis: 2HB) is described hereinafter.

An automatic pure bending tester manufactured by Kato Tech Co., Ltd. (model name KES-FB2-AUTO-A) was used as a measuring device. A size of a sample used for the measurement was adjusted to 100 mm by 100 mm (in a case where a sufficient width cannot be obtained, the width was adjusted to between 10 mm to 100 mm by 10 mm intervals).

The sample is bent toward an obverse side to a maximum curvature of 2.5 cm⁻¹, by pure bending that maintains an arc on one side, with a chuck interval of 1 cm and released. Thereafter, the specimen is bent toward a reverse side to a maximum curvature of -2.5 cm⁻¹ and released. Then a relation between a curvature and a bending moment is evaluated.

The flexural rigidity (B) is obtained as a value on a hysteresis curve, and is represented by an average tilt angle of curvatures of from 0.5 to 1.5 cm⁻¹.

The flexure recovery (2HB) is represented by a hysteresis width of a bending moment M at a curvature of 1.0 cm⁻¹. Greater flexure recovery (2HB) signifies worse (lower) flexure recovery.

### 1.7. Base Absorbent Core

As shown in FIGS. 1, 3 and 4, the base absorbent core 2 includes a liquid-permeable top sheet 27, a liquid-impermeable back surface sheet 29, and a liquid retentive absorbent core 28 disposed between the top sheet 27 and the back surface sheet 29. Substantially in a center in the width direction WD of the base absorbent core 2, a belt-shaped central portion 20 is formed along the longitudinal direction LD. The top absorbent core 3 is disposed in the central portion 20. In addition, gathers 21A and 21B are formed on both sides in the width direction WD of the central portion 20. The gathers 21A and 21B are brought into contact with the groin and inhibit leakage of menstrual blood and the like not being absorbed by the top absorbent core 3 and running along the body surface. Furthermore, wings 23A and 23B are formed on both sides in the width direction WD of the absorbent article 1, so as to project outwardly in the width direction WD.

In addition, as shown in FIG. 2, substantially in a center in the width direction WD of a reverse side, being another side, of the base absorbent core 2, a dislocation preventing portion 26 is disposed. On a reverse side of the wings 23A and 23B, wing dislocation preventing portions 25A and 25B are disposed.

The central portion 20 is formed in a belt-like shape substantially in a center in the width direction WD of the base absorbent core 2 along the longitudinal direction LD. The central portion 20 is a thin region composing the base absorbent layer. The central portion 20 is composed of the top sheet 27, the back surface sheet 29, and the absorbent core 28. The top sheet 27 and the absorbent core 28 are adhesively joined by a hot melt adhesive and further joined by a plurality of compressed grooves 22 formed along the longitudinal direction LD.

In the present embodiment, the top sheet 27 of the central portion 20 is composed of an air-through nonwoven fabric having a basis weight of 30 g/m². The nonwoven fabric is formed of a fiber of 2.2 dtex and 51 mm in fiber length, with a core of polypropylene and a sheath of polyethylene. It should be noted that a hydrophilic oil solution is preferably applied on a surface of the fiber.

The absorbent core 28 is formed by laminating pulverized pulp with a high absorbance polymer blended thereinto, and wrapping the laminate with a tissue (not shown). The tissue is formed, for example, to be 15 g/m². As shown in FIG. 6, the pulverized pulp is preferably disposed so as to have different basis weights in different parts of the central portion 20. For example, in a peripheral region 201 including a position Z, the pulverized pulp can be laminated so as to be 500 g/m². Additionally, in a region 202 on both sides of the region 201 in the longitudinal direction LD, the pulverized pulp can be laminated so as to be 100 g/m². Furthermore, in a region 203 formed along the longitudinal direction LD, to the side of a rear edge 230 in the region 201, the pulverized pulp also can be laminated so as to be 100 g/m². In addition, to the side of a front edge 220 in the longitudinal direction LD in the region 201, a region 204 is formed where the pulverized pulp is 300 g/m². In another region 205, the pulverized pulp can be laminated so as to be 200 g/m². In addition, the high absorbance polymer is blended into the pulverized pulp so that an average basis weight of the entire absorbent core 28 is 10 g/m²; however, in each portion in the absorbent core 28, the high absorbance polymer is dispersedly disposed proportionately with the basis weight of the pulverized pulp. This is because the high absorbance polymer is dispersed uniformly in the pulverized pulp.

As shown in FIG. 7, a compressed groove 22 is formed in the central portion 20. The compressed groove 22 is formed by alternately disposing high-compression portions and low-compression portions in the longitudinal direction LD. In addition, the compressed groove 22 is formed from the front region FA to the rear region BA of the central portion 20. A length in the width direction WD of the compressed groove 22 is, for example, 2 mm. In addition, a distance in the width direction WD between adjacent compressed grooves 22 is, for example, 4 mm.

As shown in FIG. 7, six compressed grooves are formed in the front region FA. On the outside in the width direction WD, a compressed groove 223 (described later) is formed. The compressed groove 223 is described later. Compressed grooves 221, 221 are formed inside in the width direction WD of the compressed grooves 223 and 223. The compressed grooves 221 and 221 are formed from the front region FA to the rear region BA and connected to each other in the rear region BA. The compressed grooves 221 and 221 thus form a U-shaped continuous compressed groove projecting toward the rear edge 230. Compressed grooves 222 and 222 are formed inside in the width direction WD of the compressed grooves 221 and 221. The compressed grooves 222 and 222 are linearly formed from the front region FA to a central region CA.

In the central region CA, in addition to the six compressed grooves 221, 222, and 223, an end portion of a compressed groove 224 (described later) is formed. The compressed groove 223 is formed from the front region FA to a central region CA, in a slightly curved shape projecting inward in the width direction WD. The compressed grooves 223 and 223 are formed so that a distance between each other is smallest in a position corresponding to the position Z in the longitudinal direction LD. In this position, the abovementioned six compressed grooves 221, 222, and 223 are formed at substantially regular intervals in the width direction WD. The compressed groove 24 is described later.

In the rear region BA, in addition to the compressed groove 221 continuously formed from the abovementioned front region FA, the compressed groove 224, a compressed groove 226, and compressed grooves 227 and 227 are formed.

The compressed groove 224 is formed outside the compressed groove 221. More specifically, the compressed groove 224 is formed so as to surround the compressed groove 221 formed in a U-shape.

The compressed groove 226 is formed inside the compressed groove 221 so as to extend in the width direction WD. More specifically, the compressed groove 226 is formed so as to connect the compressed grooves 221 and 221, which are spaced apart from each other in the width direction WD, in a position in the rear region BA in the vicinity of the central region CA. In the compressed groove 226, a curve projecting toward the rear edge 230 in the longitudinal direction LD is formed to be slightly U-shaped.

The compressed grooves 227 and 227 are formed so as to extend in the longitudinal direction LD, in a region between the compressed groove 221 and the compressed groove 226.

In the rear region BA, three compressed grooves are transversely formed in the width direction WD. In other words, in a center in the width direction WD, the three compressed grooves are formed in the longitudinal direction LD.

These compressed grooves 22 are integratedly formed by compressing the absorbent core 28 and the top sheet 27, thus providing a predetermined rigidity in the central portion 20. Additionally, this can prevent dislocation of a position of the top absorbent core 3 in a case where the absorbent article 1 is fixed to underwear and pulled up, in relation to rigidity of the top absorbent core 3. In other words, in a case where the rigidity of the base absorbent core 2 is too low with respect to the rigidity of the top absorbent core 3, the top absorbent core 3 cannot follow the curvature of the base absorbent core 2, and thus can prevent dislocation of the position of the top absorbent core 3.

As shown in FIGS. 1, 3A, 3B and 3C, the gathers 21A and 21B are disposed along the longitudinal direction LD on both sides of the central portion 20. The gathers 21A and 21B are formed so that at least a part thereof is raised in a thickness direction.

The gathers 21A and 21B of the present embodiment are disposed along the longitudinal direction LD, each in a position 64 mm spaced apart from a center in the width direction WD of the central portion 20. The gathers 21A and 21B are fixed at feet thereof, and have end portions 215A and 215B that can be displaced in the thickness direction. The end portions 215A and 215B are in contact with the groin of the wearer in a state of wearing.

The gathers 21A and 21B are portions obtained by adhesively folding a nonwoven fabric of a predetermined size into two, the nonwoven fabric having a greater hydrophobic property than the top sheet 27 of the central portion 20. Elastic stretchable members 213A and 213B are disposed between (inside) the folded nonwoven fabric.

In the present embodiment, three elastic stretchable members are disposed at substantially regular intervals, in each of the elastic stretchable members 213A and 213B. The elastic stretchable members 213A and 213B are formed of, for example, a rubber thread of 470 dtex and fixed in a state of being expanded 1.3 times thereof. The stretching force of the elastic stretchable members 213A and 213B can raise the gathers 21A and 21B.

Both end portions in the longitudinal direction LD of the gathers 21A and 21B are folded back outwardly in the width direction WD and fixed by hot melt adhesives 214A, 214B, 214C, and 214D. Additionally, an inner end portion in the longitudinal direction LD of the hot melt adhesives 214A, 214B, 214C, and 214D becomes the feet of the gathers 21A and 21B, and a region between the feet is a portion that is raised during wearing.

In addition, the gathers 21A and 21B can be formed of, for example, a spun-bonded nonwoven fabric of 22 g/m² in basis weight, which is formed of a fiber with a core of polypropylene and a sheath of polyethylene.

The size of the gathers 21A and 21B can be changed appropriately in accordance with size of the base absorbent core 2. In addition, stress of the elastic stretchable members 213A and 213B can be determined in accordance with diameter, number, and enlargement ratio of the rubber thread used; however, the stress can also be changed appropriately in accordance with the size of the base absorbent core 2. In such a case, it is important to avoid a rough sensation of the gathers 21A and 21B when in contact with the wearer's skin. Regarding cross-sectional shape in the width direction WD of the gathers 21A and 21B, in the first embodiment, free ends of the gathers 21A and 21B are disposed outwardly in the width direction WD of the base absorbent core 2, as shown in FIG. 3B.

More specifically, by inwardly bending in the width direction WD the feet of the gathers 21A and 21B that are fixed, and by outwardly bending in the width direction WD a central portion in the longitudinal (height) direction of the gathers 21A and 21B, the end portions of the gathers 21A and 21B are disposed outward in the width direction WD. In the present embodiment, the gathers 21A and 21B are configured by folding once, without being limiting thereto. For example, by folding the gathers 21A and 21B three times, the cross-sectional shape of the free ends can be substantially a sigma-shape, projecting outward in the width direction WD of the base absorbent core 2. Alternatively, the gathers 21A and 21B can be disposed with the free ends facing inwardly in the width direction WD of the base absorbent core 2.

In addition, starting points of standing of the gathers 21A and 21B are preferably not in the same position as the end portion to the side of the rear edge of the fixing portion 4 in the longitudinal direction LD. More preferably, the end portion to the side of the rear edge 230 of the fixing portion 4 is disposed more to the side of the rear edge 230 than the starting points of uprise of the gathers 21A and 21B. This is because, since the starting points of uprise of the gathers 21A and 21B are portions subjected to a stretching force of the elastic stretchable members 213A and 213B and the end portion to the side of the rear edge 230 of the fixing portion 4 is a portion subjected to a force when the top absorbent core 3 is spaced apart from the base absorbent core 2, applying the two forces to the same position should be avoided.

In addition, the back surface sheet 29 can be disposed on a side of the base absorbent core 2 opposite to the side where the top absorbent core 3 is disposed, the back surface sheet 29 being a liquid impermeable sheet. By disposing the back surface sheet 29 being a liquid impermeable sheet, it is possible to prevent a predetermined liquid not fully absorbed by the base absorbent core 2 from contacting underwear.

The wings 23A and 23B are formed on both sides in the width direction WD of the absorbent article 1, so as to project outwardly in the width direction WD. The wings 23A and 23B are composed of the back surface sheet 29 being a liquid permeable sheet and the nonwoven fabric constituting the gathers 21A and 21B, and formed so as to project outwardly in the width direction WD of the base absorbent core 2. The back surface sheet 29 and the nonwoven fabric are adhesively joined by a hot melt adhesive. As shown in FIG. 2, wing dislocation preventing portions 25A and 25B are disposed on an underwear side of the wings 23A and 23B. Thus, by folding back the wings 23A and 23B toward the underwear and sticking the wing dislocation preventing portions 25A and 25B to the underwear, the entire absorbent article 1 can be fixed to the underwear.

For example, 110 mm can be exemplified for a distance from the position Z to front edge 220, which is a front edge portion in the longitudinal direction LD. In addition, a distance from the position Z to the rear edge 230 can be exemplified similarly at 220 mm.

Here, a length in the width direction WD of the central portion 20 of the base absorbent core 2 is, for example, 70 to 120 mm except for the wings 23A and 23B, and is preferably 80 to 110 mm. In the first embodiment, 110 mm can be exemplified. In addition, a length in the width direction WD of the base absorbent core 2 including the wings 23A and 23B is 120 to 200 mm, and preferably 140 to 180 mm. In the first embodiment, 160 mm can be exemplified for a length in the width direction WD including the wings 23A and 23B. The length of the base absorbent core 2 in the width direction WD is preferably 1.5 to 7 times, and more preferable 2 to 5 times, a width of the top absorbent portion 30.

A length in the longitudinal direction LD of the base absorbent core 2 is, for example, preferred to be 250 to 500 mm, and more preferably 270 to 450 mm. Additionally, in the first embodiment, 330 mm can be exemplified for a length in the longitudinal direction LD. In addition, a length in the width direction WD of the base absorbent core 2 except for the wings 23A and 23B is preferred to be 70 to 120 mm, and more preferably 80 to 110 mm.

The absorbent article 1 can be produced by, for example, a manufacturing process including the following procedures. Initially, as for the base absorbent core 2, the top sheet 27 and the absorbent core 28 are arranged to be layered and the laminate is pressure-bonded by way of an embossing roller with a predetermined pattern from a side of the absorbent core 28, thus forming the compressed groove 22 and pressure bonding the top sheet 27 and the absorbent core 28. Furthermore, a liquid impermeable back surface sheet 29 is arranged to be layered and adhesively joined by a hot melt adhesive on a side of the absorbent core 28 opposite to the top sheet 27, thus forming a base absorbent core 2.

In addition, as for the top absorbent core 3, the top absorbent portion 30 is formed by disposing thereof in a predetermined position and wrapping with the top sheet 331 the absorbent core 35 of an elongated shape, the second sheet 332, and the back surface sheet 34. Then, the handle portion 40 is formed by performing an embossing process on a region in one of both end portions in the longitudinal direction LD where the absorbent core 35 is not disposed. In addition, the other end is adhesively joined to the front region FA of the base absorbent core 2 by applying a hot melt adhesive. As a result, a region where the top absorbent core 3 and the base absorbent core 2 are joined (joining portion 47) and the pressure-bonding portion 46 (later described) form the fixing portion 4, and a side of the top absorbent core 3 where the handle portion 40 is formed becomes the free end 31.

Furthermore, the temporary fixing portion 5 is formed by performing heat-treatment by way of embossing a region where a region on the top absorbent core 3 to the side of the free end 31, formed only of the top sheet 331 and the back surface sheet 34, and a region on the base absorbent core 2 to the side of the rear edge 230, formed only of the top sheet 27 and the back surface sheet 29, overlap each other. In addition, at the same time, the top absorbent core 3 is fixed on the base absorbent core 2, by performing heat-treatment by way of embossing a region where a region on the base absorbent core 2 to the side of the front edge 220, formed only of the top sheet 27 and the back surface sheet 29, and the top absorbent core 3 overlap each other.

### 1.8. Mode of Usage

An example of a mode of usage of the absorbent article 1 is described hereinafter with reference to FIGS. 8 to 10.

First, as shown in FIG. 8, the absorbent article 1 is in a state where the top absorbent core 3 is disposed on the base absorbent core 2. The top absorbent core 3 is disposed along the longitudinal direction LD in a central portion 20 of the base absorbent core 2. In addition, the top absorbent core 3 is temporary fixed on the base absorbent core 2 by way of the temporary fixing portions 5 and 5. The whole absorbent article 1 is configured to be deformable into a smooth U-shape as a whole, so as to follow a curvature in the vicinity of an excretory part of a wearer's body.

Next, as shown in FIG. 9, the absorbent article 1 is disposed on a crotch part of the underwear. Additionally, similarly to a conventional absorbent article, the wings 23A and 23B are folded back so as to wrap around a corresponding portion of the underwear. The wing dislocation preventing portions 25A and 25B disposed on a reverse side of the wings 23A and 23B fixes the absorbent article 1 on the underwear. Thereafter, in a state where the top absorbent core 3 is disposed on an upper face of the base absorbent core 2, the absorbent article 1 is pulled up toward a wearer's body along with the underwear. Here, due to the top absorbent core 3 being in a state temporarily fixed by way of the temporary fixing portion 5, the top absorbent core 3 is not spaced apart from the base absorbent core 2 until the temporary fixing is released in a wearing process.

Subsequently, as shown in FIG. 10, the wearer inserts a hand from the back of the body, grips the handle portion 40, and pulls up the top absorbent core 3 in a direction indicated by an arrow R.

As a result, the top absorbent core 3 is spaced apart from the base absorbent core 2, being released from temporary fixing by the temporary fixing portion 5. The top absorbent core 3 being spaced apart from the base absorbent core 2 is displaced so as to slip into the gluteal cleft, from the fixing portion 4 as a starting point. In other words, the top absorbent core 3 is spaced apart from the base absorbent core 2 by the handle portion 40 gripped and pulled up by the wearer. Additionally, the top absorbent core 3 is in contact with an excretory part and displaced so as to slip into the gluteal cleft.

Here, the top absorbent core 3 in contact with an excretory part and disposed so as to slip into the gluteal cleft deforms so as to follow a shape of an excretory part and gluteal cleft. More specifically, since the flexural rigidity of the top absorbent core 3 is 0.05 to 1.7 N, the top absorbent core 3 is easily deformed to follow the vicinity of an excretory part and the gluteal cleft.

Furthermore, since a compression resilience ratio (RC) of the top absorbent core 3 is at least 30%, the top absorbent core 3 deformed and slipped into the gluteal cleft can restore to the original shape easily, and can keep conformability thereof even after readjusting a position of the top absorbent core 3.

In addition, since the difference in the flexural rigidity between adjacent portions is no greater than 1.2 N, the top absorbent portion 30 does not have a portion causing an extreme difference in rigidity in longitudinal direction LD. As a result, the top absorbent core 3 can be flexibly deformed along a wearer's body from the front edge 220 to the rear edge 230 thereof.

In addition, since the absorbent core 35 of the top absorbent core 3 has different basis weights in different portions, a portion having a lower basis weight than the periphery thereof becomes a fold starting point and allows the top absorbent core 3 to be deformed to follow a shape of the excretory part and the gluteal cleft. More specifically, a region 357 formed substantially in a center in the width direction WD of the top absorbent portion 30 is disposed so as to have a lower basis weight than the periphery thereof. As a result, the region 357 becomes a fold starting point and allows the top absorbent core 3 to be deformed into a mound shape with the region 357 as an apex, so as to slip into the gluteal cleft.

The region 357 is formed to correspond to a region from the perineal area to the anus when the top absorbent core 3 is in contact with the gluteal cleft. As a result, the top absorbent core 3 is formed to be able to reach the deepest area in the gluteal cleft. Thus, since the top absorbent core 3 deforms in accordance with a shape of the excretory part and the like, the top absorbent core 3 can be disposed to adhere to the excretory part and the like. The top absorbent core 3 is disposed to adhere to a wearer's body without a gap. In other words, the top absorbent core 3 is disposed to be in contact with the excretory part to directly absorb menstrual blood and the like, and to inhibit leakage of menstrual blood and the like running along a surface of a wearer's body.

In addition, the base absorbent core 2 absorbs a predetermined liquid that is not fully absorbed by the top absorbent core 3. The liquid not fully absorbed by the top absorbent core 3 is absorbed by the absorbent core 28 in the base absorbent core 2 via a portion where the top absorbent core 3 and the base absorbent core 2 are in contact with each other. The predetermined liquid absorbed by the base absorbent core 2 is prevented from contacting the underwear by the liquid impermeable back surface sheet 29 and retained by the absorbent core 28 in the base absorbent core 2.

Here, although a case of using the absorbent article 1 with the top absorbent core 3 spaced apart from the base absorbent core 2 has been described above, the absorbent article 1 can be used with the top absorbent core 3 not spaced apart from the base absorbent core 2. In other words, the absorbent article 1 can be used in the same mode as with a conventional sanitary napkin, by not releasing the temporary fixing by the temporary fixing portion 5. The wearer can choose which mode of usage the absorbent article 1 is used when wearing.

According to the present embodiment, since the top absorbent core 3 is spaced apart from the base absorbent core 2 with the fixing portion 4 as a starting point, the top absorbent core 3 can be manipulated independently from the base absorbent core 2. As a result, a degree of freedom in motion of the top absorbent core 3 can be increased and the absorbent core can adhere to a wearer's body continuously without being affected by the motion of clothing on which the base absorbent core 2 is disposed.

In the present embodiment, since compression hardness of the top absorbent core 3 is 0.1 to 2 times that of the base absorbent core 2, and a difference in rigidity between the top absorbent core 3 and the base absorbent core 2 is small, the top absorbent core 3 can be prevented from sinking into the base absorbent core 2 when a pressure is applied to the top absorbent core 3 or to the base absorbent core 2.

In the present embodiment, since the top absorbent core 3 and the base absorbent core 2 are joined to each other by the fixing portion 4, the top absorbent core 3 and the base absorbent core 2 are not completely separated from each other. Therefore, when the wearer removes the absorbent article from the body, the top absorbent core 3 can be removed only by pulling down the underwear to which the base absorbent core 2 is fixed.

In the present embodiment, due to an appropriate flexural rigidity, the top absorbent core 3 can adhere to the gluteal cleft of complex shape. Furthermore, due to an appropriate compression hardness and an appropriate compression resilience ratio, adhesion and conformability to a wearer's body can be maintained even in a case where a pressure is applied to the top absorbent core 3 and/or the base absorbent core 2, and feeling of discomfort, in a case where the top absorbent core 3 is in contact with a wearer's body, can be lessened.

In the present embodiment, the flexural rigidity of the top absorbent portion 30 within a predetermined range allows the top absorbent portion 30 to be in conformable contact with a wearer's body. In addition, since a basis weight of the absorbent core 35 is in a range of 300 to 500 g/m² while the top absorbent portion 30 is soft and in conformable contact with a wearer's body, a sense of security when wearing can be provided.

In the present embodiment, the top absorbent core 3 can be deformed at a portion of different basis weight, due to a difference in basis weight in the absorbent core 35. More specifically, the top absorbent core 3 can be deformed along a shape of the excretory part and the gluteal cleft. The top absorbent core 3 can be deformed so as to adhere to an excretory part such as the vaginal opening. In particular, by forming a low basis weight portion substantially in a center in the width direction WD of the top absorbent portion 30, the top absorbent portion can be prevented from deforming by being folded at a position other than the center in the width direction WD. Furthermore, since the low basis weight portion is formed at least from the perineal area to the anus in the longitudinal direction LD, the top absorbent core 3 can reach deep down into the gluteal cleft, which needs adhesion the most, thus avoiding leakage of liquid running on a surface of a wearer's body.

### 2. Other Embodiments

Second to sixth embodiments of the present invention are described hereinafter with reference to FIGS. 15 to 19. The second to the fifth embodiments are other embodiments including an engaging portion, and the sixth embodiment is another embodiment without the fixing portion. It should be noted that, in the following description, the same reference numerals have been retained for similar parts that are identical to that described in the first embodiment, with the descriptions thereof omitted.

### 2.1. Second embodiment

As shown in FIGS. 15A and 15B, an absorbent article 1A of the second embodiment is different from the first embodiment in that an engaging portion is provided.

The absorbent article 1A includes an engaging portion 37A as an engaging means, and a central portion 20 if the base absorbent core 2 as a target object. The engaging portion 37A is disposed to the side of the free end 31 of the top absorbent core 3 and engages the side of the free end 31 of the top absorbent core 3 with a predetermined position. More specifically, the engaging portion 37A engages the side of the free end 31 of the top absorbent core 3 with the predetermined position in a state of being worn on the wearer's body, which is a target for application.

The engaging portion 37A is disposed to the side of the free end 31 of the top absorbent core 3. More specifically, the engaging portion 37A is disposed on a top absorbent core 3A to the side of the base absorbent core 2, in a rear region BA.

In detail, the engaging portion 37A is disposed between the nondense portion 351 of the absorbent core 35 disposed substantially in a center in the longitudinal direction LD of the top absorbent portion 30 of the first embodiment, and the nondense portion 352 disposed to the side of the rear region BA. In further detail, an end portion of the engaging portion 37A to the side of the rear edge 230 corresponds to the region 355, and is disposed in the vicinity of an end portion of the nondense portion 352 to the side of the front edge 220.

The region 357 formed substantially in a center of the top absorbent portion 30 is not continuously formed to the nondense portion 352. This can maintain a region in which the engaging portion 37A is disposed to be substantially planar, since a region of different basis weight is not formed substantially in a center thereof in the width direction WD. In other words, the engaging portion 37A is disposed in a region that is not deformed into a convex shape with an apex substantially at a center in the width direction WD of the top absorbent portion 30.

In addition, the engaging portion 37A is disposed in the vicinity of the handle portion 40. The engaging portion 37A is disposed at a position that is displaced in accordance with the displacement of the handle portion 40. In other words, the handle portion 40 is disposed at a position where the displacement (position) of the engaging portion 37A can be appropriately adjusted.

Disposition of the engaging portion 37A is adjusted in accordance with a target object to engage therewith. In a case where the engaging portion 37A is engaged with the underwear as clothing, which is disposed so as to cover the absorbent article 1A, the engaging portion 37A can be disposed in the vicinity of the free end 31 or in the handle portion 40. Alternatively, in a case where the engaging portion 37A is engaged with the wearer's body, the engaging portion 37A can be disposed in the vicinity of the free end 31 on the top absorbent portion 30 or on the handle portion 40, on the skin contacting side of the top absorbent portion 30.

It should be noted that the engaging portion 37A can be disposed both on the side of the top absorbent portion 30 and on the side of the free end portion 32. In such a case, the wearer can choose whether the engaging portion 37A is engaged to the underwear, or to the body, when wearing. In addition, as described above, the disposition and number of the engaging portions 37A can be appropriately adjusted. Alternatively, although a configuration in which the engaging portion 37A is engaged with the base absorbent core 2 is described in the above description, the engaging portion 37A can be still engaged with the underwear in such a configuration.

As a member for forming the engaging portion 37A, any suitable member can be chosen in accordance with a supposed target objects. For example, in a case where it is supposed that the engaging portion 37A is engaged with the wearer's body, an adhesive is preferably used. The adhesive used can be exemplified by a hot melt adhesive and a gel adhesive.

Alternatively, in a case where it is supposed that the engaging portion 37A is engaged with the underwear or the base absorbent core 2, a hook material can be used in addition to the abovementioned adhesive as the member (material) for the engaging portion 37A. In a case where the hook material is used as the member for the engaging portion 37A, the hook material can be engaged with and fix any target object disposed in a portion corresponding to a position engaged with the engaging portion, underwear, and fiber in a surface member of the base absorbent core 2.

As the hook material, the male material of a hook and loop fastener can be exemplified. In the present embodiment, a plurality of hooks of the hook material to be engaged with fibers preferably has a predetermined directionality. "To have directionality" indicates that the plurality of hooks of the hook material is fixed on a base material of the hook material in a state of being tilted to a predetermined angle.

A hook material having directionality is engaged to a target object being pressed thereagainst. The hook material limits displacement toward a tilted direction of the hooks, and does not limit displacement toward a direction opposite to the tilted direction of the hooks. In other words, the hook material is not displaced when pulled toward a tilted direction of the hooks as a predetermined direction, and the hook material is disengaged and displaced when horizontally displaced toward a direction opposite the tilted direction of the hooks as the predetermined direction.

In a case of using the hook material having directionality as the engaging portion 37A, the hook material is disposed so as the tilted direction of the hooks is toward the fixing portion 4 of the top absorbent core 3. Therefore, when the top absorbent core 3 and the engaging portion 37A are in contact with the base absorbent core 2, a tension generated by a curve of the base absorbent core 2 pulls the engaging portion 37A toward the tilted direction of the hooks, thus engaging the engaging portion 37A and limiting displacement. Alternatively, when the top absorbent core 3 is pulled outwardly, the engaging portion 37A is pulled toward the direction opposite the tilted direction of the hooks, thus disengaging the engaging portion 37A and allowing for displacement.

It should be noted that the engaging portion 37A preferably has an engaging force that is unchanged even after being attached and detached for a plurality of times. In a region in the base absorbent core 2, which is intended to contact the engaging portion 37A, a target object, which is suitable for the member used in the engaging portion 37A, can be disposed. This can prevent degradation in the engaging force after multiple attachments and detachments of the engaging portion 37A. For example, a mould releasing film can be disposed in a case where an adhesive is used for the engaging portion 37A. By bringing the engaging portion 37A into contact with the mould releasing film before use, degradation in adhesive force, due to fibers and the like adhering to the engaging portion 37A, can be avoided.

Alternatively, in a case where a hook material is used for the engaging portion 37A, the female material of the hook material, for example, can be disposed in a region in the base absorbent core 2 contacting the engaging portion 37A before use. The engaging force of the hook material is not likely to be degraded after multiple cycles of attachment and detachment; however, the hooks constituting the hook material may detach, and fibers contacting the hook material may be damaged. By disposing fuzzy loops of the hook material in a region contacting the engaging portion 37A before use of the absorbent article 1A, the hooks of the hook material falling out and damaging fibers in the region contacting the hook material can be prevented.

In addition, the engaging portion 37A can act also as a temporary fixing means, in addition to the temporary fixing portions 5 and 5. In other words, the engaging portion 37A is engaged with a predetermined position in the base absorbent core 2A before use of the absorbent article 1A, and limits mobility of a free end portion 32A. Alternatively, both the engaging portion 37A and the temporary fixing portions 5 and 5 can be provided.

As shown in FIG. 15B, the engaging portion 37A can be disposed in the handle portion 40A to the side of the top absorbent portion 30A, without being limiting thereto.

Here, a joining force of the temporary fixing portion 5 is defined to such a degree that the free end 31 of the top absorbent core 3 and the base absorbent core 2 is not easily spaced apart from each other, in a process of putting onto the body. Furthermore, the joining force is of a degree easily releasable by the wearer without a complex operation.

In addition, the engaging portion 37A can act also as the temporary fixing portion 5. In other words, by engaging the engaging portion 37A with the base absorbent core 2 before use, the engaging portion 37A can act as the temporary fixing portion 5. Thus, by engaging the engaging portion 37A acting as the temporary fixing portion with the base absorbent core 2, mobility of the free end 31 of the top absorbent core 3 can be limited.

The top absorbent core 3, which is disposed so as to slip into the gluteal cleft in a process of putting on the absorbent article 1A, is engaged with a surface of the base absorbent core 2 by means of the engaging portion 37A disposed to the side of the free end 31. This can maintain the top absorbent core 3 in a state and position adjusted by a wearer. More specifically, since the top absorbent core 3 is brought into contact with a wearer's body having a predetermined tension produced by the fixing portion 4 and the engaging portion 37A, a predetermined force toward a wearer's body is always applied to the top absorbent core 3. In other words, the top absorbent core 3 is fixed so as to maintain a state of being in contact with the wearer's body.

In addition, by using a hook material having directionality for the engaging portion 37A, s position of the top absorbent core 3 can be readjusted even after engaging the top absorbent core 3 as described above. In other words, the wearer can disengage the engaging portion 37 and restore mobility, only by gripping the handle portion 40 from the back of the body and pulling toward the rear edge 230 of the base absorbent core 2. Thereafter, the wearer can readjust the position of the top absorbent core 3 and engage the top absorbent core 3 as described above.

The position of the top absorbent core 3A adjusted when putting on can be maintained by engaging the engaging portion 37A with the base absorbent core 2, underwear, or the wearer's body, at the time of putting on. This allows the top absorbent core 3 to maintain a state of adhering to the wearer's body. Thus, in addition to appropriately absorbing liquid such as menstrual blood discharged from an excretory part, the top absorbent core 3 can inhibit leakage of liquid running along the wearer's body.

### 2.2. Third Embodiment

As shown in FIGS. 16A and 16B, an absorbent article 1B of the third embodiment is different from the first embodiment in the engaging means.

The engaging means for engaging the base absorbent core 2B with the top absorbent core 3B includes an engaging portion 37B and a target object 8B, the engaging portion 37B being a top absorbent core engaging portion disposed on the top absorbent core 3B and the target object 8B being a base absorbent core target object disposed on the base absorbent core 2B, to the side of the rear edge 230B.

The target object 8B is disposed on a face of the base absorbent core 2B facing the top absorbent core 3B, to the side of the free end 31 of the top absorbent core 3B. More specifically, the target object 8B is disposed so as to cover the central portion 20, in the vicinity of the end portion of the base absorbent core 2B to the side of the rear edge 230. Additionally, both side portions of the target object 8B in the width direction WD of the base absorbent core 2B are fixed to the base absorbent core 2B.

A length of the target object 8B in the longitudinal direction LD of the base absorbent core 2B is preferably at least 2 cm, and more preferably 3 to 15 cm. In a case where the length in the longitudinal direction LD of the target object 8B is smaller than 2 cm, a region able to contact the engaging portion 37B is small and engagement may not be obtained preferably when a wearer adjusts the position of the top absorbent core 3B and engages the engaging portion 37B with the target object 8B by overlapping. In addition, a length of the target object 8B in the width direction WD of the base absorbent core 2B is preferably 2 to 15 cm, and more preferably 3 to 8 cm. In a case where the length in the width direction WD of the target object 8B is smaller than 2 cm, a region in the target object 8 able to contact the engaging portion 37B is small, and thus the engaging portion 37B and the target object 8 may not be engaged with each other when putting on. Alternatively, in a case where the length in the width direction WD of the target object 8B is greater than 15 cm, the length exceeds the width of the base absorbent core 2B.

In addition, the target object 8B is disposed in a position closer to the rear edge portion 230 than a position of the engaging portion 37B in a case where the base absorbent core 2B and the top absorbent core 3B are made to be substantially planar. More specifically, the target object 8B is disposed in a position closer to the rear edge portion 230 by at least 2%, preferably at least 5%, more preferably at least 8% of the total length of the base absorbent core 2B in the longitudinal direction LD.

A member that can be used for the target object 8B is arbitrarily selectable in combination with a member constituting the engaging portion 37B (described later). More specifically, in a case where the engaging portion 37B is formed of a hook material (described later), an elastic nonwoven fabric, a surface member similar to a liquid permeable sheet that can be used in the central portion 20 of the base absorbent core 2B, and a loop material can be exemplified as a member for the target object 8B. For the elastic member, a stretching ratio of the elastic member being 1.05 to 3 times, preferably 1.1 to 2.5 times, and more preferably 1.2 to 2.0 times is desirable.

In addition, in a case where the engaging portion 37B is formed of a hook material, the target object 8B is preferably an elastic member such as a stretchable nonwoven fabric. More specifically, the target object 8B is preferably a stretchable nonwoven fabric; however, a nonelastic member that can be engaged with the hook material may also be employed. More specifically as a member having elasticity, a member is preferred that has stretchability of approximately 3 to 50%, and more preferably 5 to 20% with respect to a distance between the fixing portion 4 and the engaging portion 37B in the longitudinal direction LD of the base absorbent core 2. By using such an elastic member, a feeling of tightness on the wearer's body can be reduced and a buffer region can be provided that prevents disengagement between the engaging portion 37B and the target object 8B due to stretching of the target object 8B in a case where the wearer's body or underwear is displaced when wearing the absorbent article 1B.

In a case where the engaging portion 37B is formed of an adhesive, a member that can be used as the target object 8B is, for example, a member to which an adhesive can be fixed, such as a mold-releasing film, and preferably a member allowing reengagement.

The engaging portion 37B is preferably disposed to the side of the free end portion 32 of the top absorbent core 3B.

The engaging portion 37B is preferably disposed in a position in the vicinity of the target object 8B, more inwardly than the target object 8B, in a case where the base absorbent core 2B and the top absorbent core 3B are made to be substantially planar. This disposition is especially preferable in a case where the target object 8B is formed of the elastic member. It should be noted that, in a case where the target object 8B is not formed of the elastic member, the engaging portion 37B and the target object 8B can be arranged so as to be overlapping.

As a member for forming the engaging portion 37A, the same member as in the second embodiment can be used. The member can be selected in combination with a member constituting the engaging portion 37B. For example, an adhesive such as a hot melt adhesive, and a member that is easily detachable such as a hook material, can be used.

In a case where a hook material having the abovementioned directionality is used for the engaging portion 37B, a nonwoven fabric, a loop material, an elastic member and the like can be exemplified as a member used for the target object 8B.

The engaging portion 37B can be disposed in a plurality of positions. In detail, for example, in addition to the engaging portion 37B disposed on the top absorbent portion 30B, the engaging portion 37B can be disposed in at least a part of the handle portion 40B. In more detail, in addition to the engaging portion 37B disposed inwardly on the top absorbent portion 30B, the engaging portion 37B can be disposed in a position corresponding to the vicinity of the rear edge 230 on the base absorbent core 2B. By providing an engaging portion in the vicinity of the free end portion 32 of the top absorbent portion 30B, a wearer can choose to engage the engaging portion with underwear when putting on the absorbent article 1B.

### 2.3. Fourth Embodiment

As shown in FIGS. 17A and 17B, an absorbent article 1C of the fourth embodiment is different from the first embodiment in regards to the engaging means.

In the fourth embodiment, an engaging portion 37C is disposed on the base absorbent core 2C. More specifically, for example, the engaging portion 37C is preferably disposed in a position 0 to 100 mm, and more preferably 20 to 80 mm, apart from the rear edge 230C of the base absorbent core 2C. The engaging portion 37C is preferably formed in a substantially elongated shape and disposed along the longitudinal direction LD of the base absorbent core 2C. Alternatively, the engaging portion 37C can be disposed so as to transverse the central portion 20C in the width direction WD.

5 to 100 mm, and preferably 10 to 80 mm, can be exemplified for a length in the longitudinal direction LD of the engaging portion 37C. In addition, 5 to 80 mm, and preferably 7.5 to 60 mm, can be exemplified for a length in the width direction WD of the engaging portion 37C.

As a member for forming the engaging portion 37C, the same member as the engaging portion 37A in the second embodiment can be used, for example.

A target object 8C is disposed on the top absorbent portion 30C, on a side facing the base absorbent core 2C. A loop material including a liquid impermeable sheet can be exemplified for the target object 8C. By forming the target object 8C with the loop material including a liquid impermeable sheet, the target object 8C can engage with the engaging portion 37C being in contact therewith, and leakage of liquid from a side of the top absorbent core 3C facing the base absorbent core 2C can be prevented.

### 2.4. Fifth Embodiment

As shown in FIGS. 18A and 18B, an absorbent article 1D of the fifth embodiment is different from the first embodiment regarding the point of providing an engaging means.

The engaging portion 37D of the fifth embodiment is disposed to the side of the free end portion 32 of the top absorbent core 3D. More specifically, the engaging portion 37D is formed along the width direction WD in the free end portion 32.

As a member for forming the engaging portion 37D, the same member as the engaging portion 37A in the second embodiment can be used. Preferably, hook material having directionality as in the second embodiment can be exemplified.

A mode of usage of the absorbent article 1D is as follows. First, the base absorbent core 2 is disposed on a crotch portion of the underwear, and then the underwear is pulled up. Next, a handle portion 40D is gripped and pulled up in a thickness direction (toward the top face in FIG. 18), thus releasing temporary fixing by the temporary fixing portions 5 and 5. The top absorbent core 3 is in contact with an excretory part and is displaced so as to slip into the gluteal cleft, while the handle portion 40 is still gripped. Additionally, the engaging portion 37D is engaged with an inner surface of clothing, maintaining a state and position adjusted by the wearer.

### 2.5. Sixth Embodiment

As shown in FIGS. 19A and 19B, the sixth embodiment is different from the first embodiment in the points of having engaging portions 37aE and 37bE and handle portions 40aE and 40bE on both ends in the longitudinal direction LD of a top absorbent core 3E, which acts as an auxiliary pad, and the top absorbent core 3E and a base absorbent core 2 being manipulatable completely independently from each other.

As shown in FIG. 19B, in the present embodiment, the engaging portions 37aE and 37bE and handle portions 40aE and 40bE are disposed on both ends in the longitudinal direction LD of the top absorbent core 3E. More specifically, one of the engaging portions 37aE and 37bE can engage with the base absorbent core 2, and the other engaging portion can be a free end with the one end as a starting point. In other words, the top absorbent core 3E does not include the fixing portion 4 as in the first embodiment, and can be completely separated from the base absorbent core 2 and used as a so-called auxiliary pad of the base absorbent core 2.

The engaging portions 37aE and 37bE detachably fix the top absorbent core 3E to the base absorbent core 2. Here, the engaging portion 37aE is disposed to the side of the front edge 220 of the top absorbent core 3E, and the engaging portion 37bE is disposed to the side of the rear edge 230 of the top absorbent core 3. It should be noted that the orientation in the longitudinal direction LD in which the top absorbent core 3E is disposed on the base absorbent core 2 is arbitrarily selected.

The engaging portion 37aE, disposed to the side of the front edge 220 of the base absorbent core 2, is disposed in a region corresponding to a region in the top absorbent core 3E fixed by the fixing portion 4 of the first embodiment, in a case where the top absorbent core 3E and the base absorbent core 2 are joined with each other. In addition, the engaging portion 37aE is formed to be a similar size to the fixing portion 4 of the first embodiment on the top absorbent core 3E.

The engaging portion 37bE is formed in an elongated shape and disposed along the longitudinal direction LD, as the engaging portion 37A in the second embodiment. It should be noted that the engaging portion 37bE can also be disposed along the width direction WD.

As a member for forming the engaging portions 37aE and 37bE, the same member as the engaging portion 37A in the second embodiment can be used.

As a mode of usage, first, the base absorbent core 2 is disposed on a crotch portion of underwear. Thereafter, the base absorbent core 2 is fixed by way of the dislocation preventing portion 26 and the wing dislocation preventing portions 25A and 25B. Next, one of the engaging portions 37aE and 37bE of the top absorbent core 3E is engaged with an arbitrary position on the base absorbent core 2. As a result, the other end becomes a free end, while the end engaged with the base absorbent core 2 is a starting point. Next, the underwear is pulled up along with the base absorbent core 2 and the top absorbent core 3E. Additionally, a position of the top absorbent core is adjusted so as to contact the gluteal cleft, by pulling the handle portion 40aE or 40bE on the free end side. Thereafter, to fix the adjusted position, the engaging portion 37aE or 37bE on the free end side is engaged with an arbitrary position on the base absorbent core 2 or on the underwear.

Alternatively, after engaging the engaging portion 37aE or 37bE on the free end side, the engaging portion that is first engaged may be disengaged in order to adjust the position of the top absorbent core 3E so as to better adhere to the gluteal cleft.

In other words, a position of the top absorbent core 3E can be adjusted by engaging one of the engaging portions 37aE and 37bE with the base absorbent core 2, and then pulling the engaging portion not engaged toward the front or rear of the wearer's body, while the engaged engaging portion is a starting point. The wearer can arbitrarily select which engaging portion is to be engaged and to be pulled. In addition, the position can be adjusted by, after engaging a first engaging portion, disengaging and pulling a second engaging portion.

In the above embodiments, the temporary fixing portion 5 is formed in the free end portion 32 in the vicinity of the handle portion 40, by a dot (circular shaped) embossing process in the first embodiment; however, the present invention is not limited thereto. For example, a plurality of linear or dot shaped embossed patterns can be continuously formed along the longitudinal direction LD, on both sides in the width direction WD of the top absorbent portion 30. Alternatively, a plurality of dot shaped embossed patterns can be formed on a whole surface of the top absorbent portion 30. Furthermore, a shape of the embossed pattern is arbitrary and not limited to a dot shape and, as in the embossing process for the handle portion 40 in the first embodiment, can be elaborately designed to have a flower shape and the like.

To perform the embossing process, fiber in a target region in the top absorbent portion 30 and the base absorbent core 2 is mildly thermally-bonded by way of an embossing member with a dot pattern; however, the processing method is not limited thereto. The target region of the embossing process may also be processed by pin-embossing, which penetrates the absorbent core 35 of the absorbent portion 30, and the absorbent core 28 of the base absorbent core 2.

In addition, although in the second embodiment, the locking portion 37A arranged in the top absorbent core 3 as locking means for locking the base absorbent core 2 and the top absorbent core 3 and the top sheet 27 in the base absorbent core 2 as a target object is described, the present invention is not limited thereto. For example, the target object and the locking portion may be respectively arranged on the side of the top absorbent core 3 and the base absorbent core 2, for example. In addition, the target portion is not limited to the surface of the base absorbent core 2, and may be a sheet-shaped member arranged on the surface of the base absorbent core 2. Furthermore, the sheet-shaped member may have stretchability. In a case where the target object is a sheet-shaped member having stretchability, it serves as a buffer region that prevents the locking portion 37 from coming off the target object because the target object is stretched when the wearer's body or the underwear moves. It should be noted that, although the hook material having directionality has been described in the description of the engaging portion 37A, the present invention is not limited thereto and a hook material formed by arranging a plurality of mushroom-shaped pins may also be used, for example.

In the second embodiment, although the locking portion 37A is locked to the base absorbent core 2 in a worn state, the present invention is not limited thereto, and may be locked to the inside of the underwear serving as a garment arranged outside the absorbent article. More specifically, the locking portion 37 is locked to an inner side surface of the underwear with the absorbent article 1 arranged between the wearer's body serving as a wearing object and the underwear serving as a garment arranged so as to cover the wearer's body. In this case, the locking portion 37 is arranged closer to the free end 31 in relation to the position where the locking portion 37 is arranged when locked to the base absorbent core 2.

In the abovementioned embodiments, although the base absorbent core 2 has the gathers 21A and 21B and the compressed grooves 22, the present invention is not limited thereto, and need not have the gathers 21A and 21B and the compressed grooves 22. In addition, although the base absorbent core 2 has the six compressed grooves 22 at substantially regular intervals in the width direction WD in the central portion 20, the present invention is not limited thereto. For example, the base absorbent core 2 may include an annular compressed groove extending in the longitudinal direction LD and formed such that a portion, corresponding to the width direction WD, of the position Z is concaved inward in the width direction WD and a gently curved compressed groove formed outside a portion formed so as to be concaved inward in the width direction WD in the annular compressed groove.

In addition, in the embodiments, although the absorbent core 28 in the base absorbent core 2 includes the nondense portions 351 and 352 and the regions 353, 354, 355, 356, and 357 respectively having different basis weights, the respective positions where they are formed and the basis weights thereof are not limited thereto, and may be formed at other positions and may have different basis weights. In addition, the absorbent core 28 may have a uniform basis weight in all the regions.

In addition, the temporary fixing portion 5 is formed by pressure bonding on the base absorbent core 2 by way of an embossing process; however, the present invention is not limited thereto. For example, the temporary fixing portion 5 may also be formed of a hot melt adhesive of low tack property such as an olefin adhesive, or of an ultrasonic seal.

In addition, the base absorbent core 2 is not limited to the abovementioned configuration thereof, and a sanitary napkin commercially available may be generally used.

In addition, a sanitary napkin commercially available may be generally used. The overall shape of the base absorbent core 2 is not limited to a substantially rectangular shape and, for example, may be a vertically-long shape such as an elliptical shape.

### 3. Example

### 3.1. First Example

A top absorbent core 3 was prepared so that a pulverized pulp with a predetermined basis weight was made 35 mm long in the width direction WD, which was wounded by an air-through non-woven fabric of 35 g/m², and the overlapped portions were adhered by a hot melt adhesive. The top absorbent core 3 was measured with respect to the flexural rigidity and compression hardness in accordance with the methods described, in a manner that the top absorbent core 3 is bent at a predetermined position in the longitudinal direction LD so as to form a broken line parallel with the width direction WD. Furthermore, the samples of "pressed" in Table 3 shown below indicate those in which the resulting top absorbent core 3 was treated by a pressure-bonding process using a press with a flat surface.

In addition, a sensory assessment test was performed on the top absorbent core 3. In the sensory assessment method, ten women having a hip size of 85 to 95 cm were requested to respectively wear respectively the samples shown below as a test to evaluate familiality with the wearer's body and a secure feeling during attachment by scoring with a maximum of ten points. The use order was random. Additionally, the results were classified based on the evaluation average of ten women, such as A: 8 or higher, B: below 8 and 6 or higher, C: below 6 and 4 or higher, and D: below 4.

**[table 1]**

| Test sample | | P1 | P2 | P3 | P4 | P5 | P6 | P7 | G |
|---|---|---|---|---|---|---|---|---|---|
| Basis Weight of Absorbent Core (Pulverized Pulp) | | 200 | 300 | 450 | 500 | 600 | 800 | 1000 | 800 |
| Press of Absorbent Core | | - | - | - | - | - | - | - | Pressed |
| Thickness | | 5.6 | 7.8 | 11.2 | 12.3 | 14 | 17.8 | 22.4 | 11.9 |
| Flexural Rigidity Value | | 0.09 | 0.16 | 0.4 | 0.48 | 0.66 | 1.2 | 1.72 | |
| Compression hardness (LC) | | 0.58 | 0.61 | 0.68 | | 0.74 | 0.73 | - | 0.83 |
| Sensory Assessment | Familiality | A | A | A | A | A | B | C | C |
| | Sense of Security | C | B | A | A | A | B | D | - |

When the top absorbent core 3 was prepared with a lower basis weight, the familiality was higher, but score for feeling was lacking, and liquid-absorbing capacity was less, resulting in a lower score for secure feeling.

When the top absorbent core 3 was prepared with a higher basis weight, the result was inferior as for familiality and secure feeling. This is because a rigid feeling was generated and user felt a gap caused by the fact that a larger material amount makes close contact with the gluteal cleft impossible.

Furthermore, the result of the familiality in the sensory assessment was such that samples P1 to P6 were favorable. In addition, the result of the secure feeling was such that samples P2 to P6 were favorable. Sample P7, of which the value of flexural rigidity was greater than 1.7 N, had an inferior evaluation result in terms of familiality as well as secure feeling. Furthermore, the sample G had a compression hardness (LC) of above 0.8 (-), but the sample G had a result of inferior familiality.

### 3.2. Second Example

A base absorbent core 2 was prepared in a way so that a pulverized pulp was wound by a tissue of 15 g/m², to which an air-through non-woven fabric of 35 g/m² was adhered by a hot melt adhesive, followed by forming a compressed groove 22 to consolidate them. Then a base absorbent core 2 was prepared by laminating an impermeable film of 23 g/m² to the back side. The length of the base absorbent core 2 was 75 mm in the width direction WD.

Furthermore, a pattern of two different compressed grooves 22 was formed. The compressed groove pattern A is similar to the pattern of the compressed groove 22 in the first embodiment. Additionally, the measurement site is a region, having a center at the position Z in the longitudinal direction LD, where six compressed grooves 22 are formed with substantially equal spacing in the width direction. Furthermore, the compressed groove pattern B is formed in the base absorbent core 2 to have a circular compressed groove that extends in the longitudinal direction LD, in which the compressed groove is formed so that the potion corresponding to the position Z in the width direction WD sinks to inside in the width direction WD as well as a shallow curved compressed groove that is formed outside the portions where the former groove is formed to sink to inside in the width direction WD. That is, four compressed grooves are formed at the measurement site in the width direction WD. Additionally, the absorbent articles with the respective compressed groove patterns were measured for their flexural rigidity in a case where the bending was performed so as to form a broken line parallel with the width direction WD. The measurement method of the flexural rigidity was as described above.

**[table 2]**

| Test sample | H1 | H2 | H3 | H4 |
|---|---|---|---|---|
| Basis weight of absorbent core (pulverized pulp) | 300 | 400 | 500 | 750 |
| Compressed groove pattern | A | A | B | B |
| Thickness of center portion | 3.26 | 3.89 | 6.14 | 10.4 |
| Flexural rigidity | 0.24 | 0.75 | 0.32 | 1.3 |

Absorbent articles 1 were prepared by a combination of samples P6 and H1 and a combination of samples P7 and H2 prepared in Example 1, and an attachment test was carried out.

The result was that the top absorbent core 3 resulted in dislocation since the base absorbent core 2 has a higher rigidity and is weaker, and thus the top absorbent core 3 cannot follow the curvature of the main body in the step of attaching the absorbent article 1 to the underwear.

### 3.3. Third Example

Test samples of the handle portion 40 were respectively produced in configurations shown in Table, described below, to perform sensory evaluations of hardness (flexural rigidity), flexure recovery, and a handhold feeling of each of the test samples.

The hardness (flexural rigidity), the flexure recovery, and the handhold feeling were evaluated by 10 women performing a sensory test. The test samples were tested in random order. The evaluation was the average of scores in a case where each of the items was evaluated on a scale of one to ten. The evaluation was represented by A, B, C, and D for an average scores of not less than 8, less than 8 to not less than 6, less than six to not less than four, and not more than four, respectively.

### Evaluating method

In a method for evaluating hardness, hardness that was preferable in gripping the handle portion 40 serving as a test sample was given a score.

Regarding the flexure recovery, the handle portion 40 serving as a test sample was folded into two and was left as it was for one hour under a temperature of 20°C and a humidity of 60% with a load of 20 g/m² applied. Thereafter, after the handle portion 40 was left as it was for one hour with the load released and further under the same humidity, such hardness that did not produce an unpleasant sensation at an end of a fold was scored.

The handhold feeling of the handle portion 40 was evaluated with regards to the thickness (mm) of the handle portion 40. More specifically, the handhold feeling in gripping the handle portion 40 was given a score depending on whether or not the holding feeling was a preferable thickness. "HMA" described in Table 3, described below, is an abbreviation of hot melt adhesives.

**[table 3]**

| | Hardness | | Resilience | | Thickness : Handhold feeling | | |
|---|---|---|---|---|---|---|---|
| Test sample | B | Sensory | 2HB | Sensory | Thickness | Sensory | Configuration |
| 1 | 0.049 | D | 0.0961 | A | 0.912 | B | An air-through nonwoven fabric having a basis weight of 35 g/m² |
| 2 | 0.1943 | B | 0.439 | A | 1.78 | A | Two sheets of air-through nonwoven fabric having a basis weight of 35 g/m² laminated with HMA |
| 3 | 0.3321 | A | 0.765 | A | 3.56 | B | Four sheets of an air-through nonwoven fabric having a bas i s weight of 35 g/m² laminated with HMA |
| 4 | 0.3565 | A | 1.0248 | A | 1.75 | A | An air-through nonwoven fabric having a basis weight of 35 g/m² and a film having a basis weight of 23 g/m² laminated with HMA and folded into three |
| 5 | 0.4507 | A | 0.5643 | A | 0.487 | C | Two sheets of SMS nonwoven fabric having a basis weight of 35 g/m² laminated with HMA |
| 6 | 0.5685 | A | 1.0863 | A | 0.741 | B | Three sheets of SMS nonwoven fabric having a basis weight of 35 g/m² laminated with HMA |
| 7 | 0.7795 | A | 2.914 | A | 0.906 | A | Four sheets of SMS nonwoven fabric having a basis weight of 35 g/m² laminated with HMA |
| 8 | 1.0865 | B | 6.3762 | B | 1.367 | A | Six sheets of SMS nonwoven fabric having a basis weight of 35 g/m² laminated with HMA |
| 9 | 1.2719 | C | 10.7168 | D | 1.567 | A | Seven sheets of SMS nonwoven fabric having a basis weight of 35 g/m² laminated with HMA |

The results were that the hardness of the handle portion 40 was preferably in a range of 0.1 to 1.2 (10⁻⁴N·m²/m). In addition, the results were that the flexure recovery of the handle portion 40 was preferably not more than 10 (10⁻²N·m/m). Furthermore, the results were that the thickness of the handle portion 40 was preferably in a range of 0.5 to 4 mm.

## Claims

1. An absorbent article comprising: a first absorbent core of substantially elongated shape;
a second absorbent core disposed on a skin contacting side of the first absorbent core, along a longitudinal direction of the first absorbent core;
and a fixing portion that fixes the first absorbent core with the second absorbent core so that at least one end in the longitudinal direction of the second absorbent core is made to be a free end,
wherein a flexural rigidity of the second absorbent core toward a side that is opposite to a side facing the first absorbent core is 0.05 to 1.7 N.

2. The absorbent article according to claim 1, wherein the flexural rigidity of a central region, which is a region in the second absorbent core from a position to be in contact with an excretory part to a position being 100 mm spaced apart from the one end of the second absorbent core, is 0.1 to 1.5 N.

3. The absorbent article according to claim 1 or 2, wherein a difference of flexural rigidity of a predetermined position on the second absorbent core and flexural rigidity of another position spaced apart from the predetermined position in the longitudinal direction is no greater than 1.2 N.

4. The absorbent article according to any one of claims 1 to 3, wherein a value of the flexural rigidity of the second absorbent core is 0.1 to 2 times compared to a value of flexural rigidity of the first absorbent core toward the second absorbent core.

5. An absorbent article comprising: a first absorbent core of substantially elongated shape;
a second absorbent core disposed on one face of the first absorbent core, along a longitudinal direction of the first absorbent core;
and a fixing portion that fixes the first absorbent core with the second absorbent core so that at least one end in a longitudinal direction of the second absorbent core is made to be a free end,
wherein a compression hardness (LC) of a surface of the second absorbent core that is opposite to a side facing the first absorbent core is 0 to 0.8 (-).

6. The absorbent article according to claim 5, wherein the compression hardness (LC) of a central region, which is a region in the second absorbent core from a position to be in contact with an excretory part to a position being 100 mm spaced apart from the one end of the second absorbent core, is 0.3 to 0.75 (-).

7. The absorbent article according to claim 5 or 6, wherein a value of the compression hardness (LC) of the second absorbent core is 0.1 to 2 times compared to a value of the compression hardness (LC) of the first absorbent core.

8. An absorbent article comprising: a first absorbent core of substantially elongated shape;
a second absorbent core disposed on one face of the first absorbent core, along a longitudinal direction of the first absorbent core;
and a fixing portion that fixes the first absorbent core with the second absorbent core so that at least one end in a longitudinal direction of the second absorbent core is made to be a free end,
wherein: the second absorbent core has a first region that is formed substantially in a center in a width direction, which is a direction orthogonal to the longitudinal direction of the second absorbent core, so as to extend in the longitudinal direction; and
the first region is different in rigidity from second regions that are formed on both sides of the first region in the width direction, so as to extend in the longitudinal direction.

9. An absorbent article comprising: a first absorbent core of substantially elongated shape;
a second absorbent core disposed on one face of the first absorbent core, along a longitudinal direction of the first absorbent core;
and a fixing portion that fixes the first absorbent core with the second absorbent core so that at least one end in a longitudinal direction of the second absorbent core is made to be a free end,
wherein: the second absorbent core includes a second absorbent layer that has liquid retention properties; and
a predetermined fold starting point element is formed substantially in a center in a width direction, which is a direction orthogonal to the longitudinal direction of the second absorbent core, along the longitudinal direction of the second absorbent core.

10. The absorbent article according to claim 9, wherein: the second absorbent layer is formed by including a hydrophilic fiber; and
in the fold starting point element, a basis weight of a first region is formed to be continuous or intermittent so as to extend in the longitudinal direction, substantially in a center in the width direction of the second absorbent layer, the basis weight in the first region is different from a basis weight of second regions, which are formed on both sides in the width direction of the first region so as to extend in the longitudinal direction.

11. An absorbent article comprising: a first absorbent core of substantially elongated shape;
a second absorbent core disposed on a first face of the first absorbent core, along a longitudinal direction of the first absorbent core; and
a fixing portion that fixes the first absorbent core with the second absorbent core so that at least one end in a longitudinal direction of the second absorbent core is made to be a free end,
wherein a core portion is disposed substantially in a center in a width direction, which is a direction orthogonal to the longitudinal direction of the second absorbent core, along the longitudinal direction of the second absorbent core.

12. The absorbent article according to claim 11, wherein the core portion is a compressed portion formed to be continuous or intermittent along the longitudinal direction of the second absorbent core, substantially in a center in the width direction of the second absorbent core.
